(19) Europäisches Patentamt / European Patent Office / Office européen des brevets

(11) **EP 1 865 315 B1**

(12) **EUROPEAN PATENT SPECIFICATION**

(45) Date of publication and mention of the grant of the patent:
**13.03.2019 Bulletin 2019/11**

(21) Application number: **06730437.8**

(22) Date of filing: **29.03.2006**

(51) Int Cl.:
*G01N 33/48* (2006.01)        *G01N 33/50* (2006.01)

(86) International application number:
**PCT/JP2006/306489**

(87) International publication number:
**WO 2006/104201 (05.10.2006 Gazette 2006/40)**

(54) **CELL IMAGE ANALYZING METHOD, CELL IMAGE ANALYZING DEVICE**

ZELLBILDANALYSEVERFAHREN, ZELLBILDANALYSEVORRICHTUNG

PROCÉDÉ D'ANALYSE D'UNE IMAGE CELLULAIRE, DISPOSITIF D'ANALYSE D'UNE IMAGE CELLULAIRE

(84) Designated Contracting States:
**AT DE DK FR GB SE**

(30) Priority: **29.03.2005 JP 2005095421**
**10.06.2005 JP 2005171208**
**14.07.2005 JP 2005205574**

(43) Date of publication of application:
**12.12.2007 Bulletin 2007/50**

(73) Proprietor: **Olympus Corporation**
**Tokyo 192-8507 (JP)**

(72) Inventors:
• **MATSUO, Yuichiro**
**Hachioji-shi**
**Tokyo, 192-0911 (JP)**
• **TAKAGI, Kosuke**
**Kawagoe-shi,**
**Saitama 3500817 (JP)**
• **SHIMADA, Yoshihiro**
**Sagamihara-shi,**
**Kanagawa 2291123 (JP)**
• **KAWAI, Tsuyoshi**
**Kobe-shi,**
**Hyogo 6580082 (JP)**

(74) Representative: **von Hellfeld, Axel et al**
**Wuesthoff & Wuesthoff**
**Patentanwälte PartG mbB**
**Schweigerstrasse 2**
**81541 München (DE)**

(56) References cited:
WO-A1-03/098210       WO-A1-2004/042392
WO-A2-02/077903       JP-A- 2001 330 604
JP-A- 2002 214 228    JP-A- 2002 312 761
JP-A- 2003 500 664    JP-A- 2004 163 201
US-A- 6 134 354       US-A1- 2001 041 347
US-A1- 2004 229 210   US-B1- 6 743 576

**Description**

Technical Field

The present invention relates to a cell-image analysis method and a cell-image analysis apparatus for performing extraction of specific data by image processing images of plant and animal cells.

Furthermore, the present invention relates to a cell-image analysis method, a cell-image analysis program, and a cell-image analysis apparatus used in pathological diagnosis of cancer etc., drug screening, and so forth in which living cells are used.

Furthermore, the present invention relates to a screening method and a screening apparatus using image processing, and in particular, to a screening method and a screening apparatus which compare changes in the morphology of cells with reference to cell images captured by a microscope.

Background Art

In the fields of biotechnology and pharmaceutical development, work is carried out to check the effect or influence of substances, such as various pharmaceuticals and drugs, on plant and animal cells or microorganisms, as well as to check the responses of plant and animal cells and microorganisms to these substances.

In particular, the task of searching for drugs that have the most effect on cells from a plurality of candidate drugs is called cell-based screening, or more simply, just "drug screening" or "cell screening". This drug screening is normally carried out visually. For example, an experimenter observes cells that have been administered a drug and cells that have not been administered anything with a microscope and counts the number of cells in the field of view. It is possible to calculate the total number of these cells or the average number of cells per field of view to measure the killing effect of the drug on the cells.

Particularly in drug screening using living cells, by applying a suitable stimulus to culture cells after administering a drug and quantifying the difference relative to cells to which no stimulus is applied, the effect of the drug can be measured. In this drug screening, each process is normally conducted by a person.

Various experiments using cancer cells are, in many cases, carried out after changing the properties by gene transfection or the like of the target cancer cells. When performing this kind of unknown manipulation on cells, however, the basic properties of the cells end up being changed in many cases. In particular, in experiments on cancer cells, it is frequently the case that the properties that characterize them as cancer cells are lost; therefore, it is necessary from the outset to check whether these cells have the properties of cancer cells.

Cells are cultured and, after a fixed period of time, it is determined whether or not they are cancer cells based on a difference in the degree of proliferation of the cells before culturing and after culturing. In other words, if the number of cells increases after a fixed period of time, they are determined as being cancerous. There are also pathological diagnoses of cancer in which tissue collected from a human body by biopsy or the like is examined to determine whether or not it contains cancer cells. For this change in the number of cells, in general, an examiner manually counts the numbers of cells before and after culturing by visual observation with a microscope or the like. In drug screening of anti-cancer drugs, for example, the efficacy of the drug, that is, its ability to suppress the proliferation, is measured by administering a drug to proliferating cancer cells and counting the number of cancer cells after the passage of a fixed period of time. The cell counting and comparison of the number of cells before and after culturing are manually performed by the examiner.

However, in drug screening, the stage where the efficacy is evaluated by observing the cells is normally realized by human operation. Therefore, in screening involving searching a large quantity of drugs, the load on the experimenter is quite large, and therefore, one challenge is to automate this process.

A known apparatus for carrying out automatic cell analysis is a flow cytometer. A flow cytometer is an apparatus in which cells are isolated by flowing them in a suspension and the viability, size, shape, and so forth of individual cells is measured. By using a flow cytometer, it is also possible to perform drug screening automatically.

Furthermore, there are also cell analysis apparatuses provided with simple image analysis software. With these apparatuses, in the same process as visual observation, an image of the cells is captured with a microscope, this image is analyzed, and the effect of the drug is measured automatically.

In an apparatus using image analysis software, because pretreatment performed by the experimenter is the same as in the case of visual observation, an advantage is provided in that the load on the experimenter is small. On the other hand, current apparatuses do not always have high image analysis precision; the precision is at a level allowing analysis using a simple single parameter as the parameter to be analyzed, such as the number of dead or living cells, the approximate size of the cells, and the positions of protrusions, as well as the average thereof.

On the other hand, techniques for acquiring cell image information (for example, cells which emit fluorescence) with a camera or the like and extracting cell outlines by image processing have been introduced (for example, see Patent Document 1 or Patent Document 2).

Patent Document 1: Japanese Unexamined Patent Application, Publication No. 2001-269195
Patent Document 2: US patent, Patent No. US 6,743,576 B1.

Disclosure of Invention

Screening using cells involves operations such as estimating drug efficacy by observing cells, recognizing cell positions, extracting features of each cell, and finally analyzing these features. The features of the cells include the size of the cells, the maximum radius, the morphology of protrusions etc., the viability of each cell, and so forth.

Cell-based screening techniques using image analysis have the following superior characteristics as compared with cell analysis in which images are not used, particularly flow cytometry etc.

(1) Morphological information of the cells can be obtained. By using morphological information such as a protrusion elongation effect, for example, it is possible to perform screening for estimating the effect.
(2) Work which the experimenter has to perform as pretreatment for conducting analysis, in other words, a procedure such as cell isolation, is not necessary, which can reduce his or her load.
(3) Since the positional relationship of the cells is known, it is possible to perform analysis in which interactions between cells, for example, the influence of local cell density at the cell perimeter on the action of a compound, are indirectly taken into account.

Thus, in order to perform efficient drug screening to reduce the load on the experimenter, it is necessary to automate the cell analysis. To automate the screening operation, first it is necessary to automate cell recognition.

To perform screening efficiently, feature parameters of the cells must be efficiently selected according to the automation. For example, when screening drugs having an effect of changing the cell size, it is not possible to find effective drugs by using the number of cells as an indicator. However, in existing cell analysis apparatuses, it is common to use apparatuses in which the feature parameters of the cells are fixed in advance.

Regarding measurement of the respective parameters, the measurement accuracy thereof is disclosed, together with a typical example, and the reliability is set for the precision in advance. However, in an actual experiment, various requirements, such as the experimental conditions, the state of the cells, and the image-acquisition conditions, differ from system to system, and therefore, the precision is also considered to differ in each experiment. If the precision turns out to be not high enough, it is necessary to change the experimental conditions. For example, the pretreatment performed on the cells before image acquisition should be changed, but a system requiring complex pretreatment is unsuitable for efficient screening. Therefore, finding suitable parameters for the measurement according to the experiments and conditions is absolutely essential for realizing accurate drug screening.

Furthermore, a notable difference compared to a cell analysis method such as flow cytometry is that microscope images of the cells are analyzed as is. Using microscope images in this way is beneficial in that the experimental procedure is close to the observation method based on direct visual observation by the experimenter and does not require a procedure of its own. In addition, performing analysis using the same image as in the visual observation provides an advantage in that it is possible to interpret the results intuitively.

The drug screening is characterized in that it is closer to the conditions inside a living organism compared with a so-called test tube experiment (in vitro) for looking at reactions of compounds only, such as intermolecular reactions.

In other words, in the drug screening, it is not necessary to isolate cells, and it is possible to observe a state in which a plurality of cells are considered to interact with each other. Therefore, it is possible to obtain information that is closer to that inside a living organism. Conversely, to obtain detailed screening results, it is necessary to perform analysis using mutual positional information of the cells.

The drug screening technique described above has the following problems:

(1) To perform screening efficiently, it is necessary to automate each process performed in screening. In particular, in an analysis portion, it is necessary to correctly analyze cell images to accurately recognize the positions of cells.
(2) It is necessary to effectively select parameters of the cells to be measured. To obtain effective screening results, it is essential to select suitable cell parameters.
(3) To correctly evaluate the drug efficacy for screening, it is necessary to perform sufficient verification of the parameters.

When visually counting the number of cells which the examiner observes with a microscope, for example, cells emitting fluorescence, there is a problem in that, not only is enormous effort required, but also counting errors and so forth occur, and it is thus not possible to perform the operation correctly. In addition, in the field of drug discovery, it is necessary to analyze a large quantity of samples (for example, 1000 samples in one day), and therefore, there is a high risk of the number of errors increasing, thus causing a decline in efficiency.

When extracting the cell outline by image processing, in order to count the number of cells, processing for recognizing more cells and so forth is necessary. In many cases, however, existing techniques have not reached a level where they can be used to count the number of cells. Therefore, there is a drawback in that, eventually, the examiner must visually count the number of cells.

Automation of the screening can be divided into a stage for capturing cell images and a stage for analyzing the captured cell images. Analysis of the cell images is performed by extracting features of the cells using a suitable image processing method and comparing feature values for cell images under different conditions.

However, because there are many instances where it is not possible to eliminate noise with processing in a program, there is a problem in that, frequently, the precision of evaluating the effect of the drug is not high.

One problem in extracting cell morphology features is accurately capturing features with good precision. Precision means, first, precision in the analysis stage for capturing; in many cases, this is originates in the analysis software program etc. In addition, there is also precision related to efficacy; this is when the feature does not represent the efficacy well, and so on. If no difference in effect appears in the features even though the drug should intrinsically have some effect, the precision can be considered to be low; however, this is precision which depends on the specifications of the experimental setup and so forth.

A first reason for the reduced precision of morphological features is that the features also include noise due to substances other than living cells being included in the cell image, followed by unsuitable definition and selection of features.

The present invention has been conceived in light of the circumstances described above, and an object thereof is to provide a cell-image analysis method and a cell-image analysis apparatus that can perform screening with superior efficiency and that are capable of performing accurate measurement of the effect of drugs according to claims 1 and 9.

A further object of the present disclosure is to provide a cell-image analysis method, a cell-image analysis program, and a cell-image analysis apparatus that can reduce examiner load and examination error, and that can accurately count the number of cells.

A further object of the present disclosure is to provide a screening method and a screening apparatus that can perform drug screening with higher precision by using an appropriate morphological feature.

A cell-image analysis method according to a first aspect of the present invention includes an image-acquisition step of capturing a plurality of cell images under different image-acquisition conditions; and an analysis step of extracting a cell position and at least one feature on the basis of the plurality of cell images and performing statistical analysis with the extracted features serving as parameters.

In the analysis step described above, parameter design may be performed on the basis of correlation of a plurality of the features, and effectiveness of a compound may be evaluated using these parameters.

In the analysis step described above, weightings of the parameters may be adjusted on the basis of the correlation.

In the analysis step described above, measurement accuracy or measurement reliability of the parameters may be evaluated on the basis of the correlations and the measurement results.

In the analysis step described above, the parameters that most effectively represent a change due to conditions of a measurement system may be selected on the basis of the evaluation of the measurement accuracy or the measurement reliability of the parameters.

In the analysis step described above, the parameters that is the most effective indicator in cell evaluation is selected on the basis of the selected parameters.

In the analysis step described above, when parameter design is performed on the basis of the correlation of a plurality of the features, and the effectiveness of a compound is evaluated using these parameters, the features of the cells may be analyzed using position information of each cell.

The position information described above may include local cell density, distance to nearest cell, and information about whether or not cells are in contact.

A cell-image analysis apparatus according to a second aspect of the present invention includes an image-acquisition unit for capturing a plurality of cell images under different image-acquisition conditions; an analysis unit for extracting a cell position and at least one feature on the basis of the plurality of cell images and for performing statistical analysis with the extracted features serving as parameters.

The analysis unit described above may perform parameter design on the basis of correlation of a plurality of the features and may evaluate the effectiveness of a compound using these parameters.

The analysis unit describe above may change weightings of the parameters on the basis of the correlation.

The analysis unit described above may evaluate measurement accuracy or measurement reliability of the parameters on the basis of the correlation and the measurement results.

The analysis unit described above may select the parameters that most effectively represent a change due to conditions of a measurement system on the basis of the evaluation of the measurement accuracy or the measurement reliability of the parameters.

The analysis unit described above may select the parameter that is the most effective indicator in cell evaluation on the basis of the selected parameters.

When the analysis unit performs parameter design on the basis of the correlation of the plurality of feature values and evaluates the effectiveness of the compound using these parameters, the analysis unit may analyze the feature of cells using position information of each cell.

The position information described above may include local cell density, distance to nearest cell, and information about whether or not cells are in contact.

A cell-image analysis method according to a third aspect of the present disclosure includes an image-information inputting step of receiving light emitted from cells at predetermined time intervals as a plurality of images; a luminance-value calculating step of calculating fractions of pixels whose luminance value in the plurality of images obtained in the image-information inputting step is a specified value or above; and a comparison calculation step of comparing the fractions of pixels having the luminance value of the specified value or above in the plurality of images, which were obtained in the luminance-value calculating step.

A cell-image analysis method according to a fourth aspect of the present disclosure includes an image-information inputting step of receiving light emitted from cells at predetermined time intervals as a plurality of images; a counting step of counting numbers of cells existing in the image, in the image information obtained in the image-information inputting step; and a comparison calculation step of comparing the numbers of cells in the plurality of images on the basis of results obtained in the counting step.

In the fourth aspect described above, preferably, the counting step compares three overlapping concentric circles, including a first circle that is smaller than a cell nucleus, a second circle that is larger than the cell nucleus, and a third circle that is larger than the second circle, with a region having a luminance value of a specified value or greater in the each image; and identifies the existence of and counts cells with the condition that all pixels inside the first circle have the luminance value of the specified value or greater, pixels having the luminance value of the specified value or greater exist in a ring-shaped region between the first circle and the second circle, and pixels having the luminance value of the specified value or greater do not exist in a ring-shaped region between the second circle and the third circle.

The third aspect or the fourth aspect described above may further include a determining step of determining the activity or degree of proliferation of the cells on the basis of the calculation results obtained in the comparison calculation step.

The cells may be classified on the basis of the activity or the degree of proliferation of the cells determined in the determining step.

The effect of a drug on the cells may be quantified on the basis of the activity or the degree of proliferation of the cells determined in the determining step.

A cell-image analysis program according to a fifth aspect of the present disclosure causes a computer to execute a luminance-value calculating step of calculating fractions of pixels with a luminance value of a specified value or greater, in information of a plurality of images obtained by acquiring light emitted from cells at predetermined intervals; and a comparison calculating step of comparing the fractions of pixels having the luminance value of the specified value or greater in the plurality of images obtained in the luminance-value calculating step.

A cell-image analysis program according to a sixth aspect of the present disclosure causes a computer to execute a counting step of counting numbers of cells existing in images, in information of a plurality of images obtained by acquiring light emitted from cells at predetermined intervals; and a comparison calculating step of comparing the numbers of cells in the plurality of images on the basis of results obtained in the counting step.

In the sixth aspect described above, preferably, the counting step compares three overlapping concentric circles, including a first circle that is smaller than a cell nucleus, a second circle that is larger than the cell nucleus, and a third circle that is larger than the second circle, with a region having a luminance value of a specified value or greater in each image; and identifies the existence of and counts cells with the condition that all pixels inside the first circle have the luminance value of the specified value or greater, pixels having the luminance value of the specified value or greater exist in a ring-shaped region between the first circle and the second circle, and pixels having the luminance value of the specified value or greater do not exist in a ring-shaped region between the second circle and the third circle.

A cell-image analysis apparatus according to a seventh aspect of the present disclosure includes an image-information inputting unit for receiving light emitted from cells at predetermined intervals as a plurality of images; a luminance-value calculating unit for calculating fractions of pixels with a luminance value of a specified value or greater in the plurality of images obtained in the image-information inputting unit; and a comparison calculation unit for comparing the fractions of pixels having the luminance value of the specified value or greater in the plurality of images obtained in the luminance-value calculating unit.

A cell-image analysis apparatus according to an eighth embodiment not forming part of the present invention includes an image-information inputting unit for receiving light emitted from cells at predetermined intervals as a plurality of images; a counting unit for counting numbers of cells existing in the images obtained in the image-information inputting unit; and a comparison calculating unit for comparing the numbers of cells in the plurality of images on the basis of results obtained in the counting unit.

A screening method according to a ninth embodiment not forming part of the present invention includes analyzing a cell image captured by a microscope; and evaluating a value parameterized with an amount of deviation of a cell outline from a circle, to serve as a morphological feature representing a morphological characteristic of each cell in the cell image; and performing drug screening on the basis of this evaluation.

In the ninth embodiment described above, as the morphological feature, a value parameterized with an amount of deviation of the cell outline from at least one of an inscribed circle and a circumscribed circle may be evaluated.

By doing so, it is possible to uniformly determine the size of the central portion of the cell, the deformation of the overall shape, the spatial extent of the cell, and so forth, and it is possible to represent the morphology of the cell effectively and with superior precision.

A screening method according to a tenth aspect of the present disclosure includes analyzing a three-dimensional image constructed from a plurality of cell images captured by a microscope; evaluating a value parameterized with an amount of deviation of a cell outline from a sphere, to serve as a morphological feature representing a morphological characteristic of each cell in the cell images; and performing drug screening on the basis of this evaluation.

In the tenth aspect of the present disclosure, as the morphological feature, a value parameterized with an amount of deviation of the cell outline from at least one of an inscribed sphere and a circumscribed sphere may be evaluated.

In the ninth aspect or the tenth aspect described above, preferably, on the basis of the morphological feature, the cells in the image are determined to be either living cells or dead cells and are used in the evaluation.

It goes without saying that, when performing this determination, the cells may be fixed prior to image capturing.

The ninth aspect or the tenth aspect described above preferably further includes performing analysis on any selected regions in the cell images; and classifying these regions as cell bodies, cell protrusions having a tubular structure, and dead cells or air bubbles, which are non-cell regions, on the basis of the parameterized value.

When performing the classifying described above, it is preferable to quantify image information for classified non-cell regions; and correct statistical results of drug screening on cells using the quantified image information for non-cell regions.

In practice, in a system in which the cells are damaged, causing some of them to die, the precision of the cell analysis increases by quantifying not only the amount, such as the total number, of living cells or dead cells that maintain their cell-like shape to some extent, but also image information of non-cell regions existing in the cell image, such as fragmented dead cells, and using this as additional information for the results of image analysis carried out on only the cells.

When quantify correcting statistical results of drug screening on cells using the quantified image information for non-cell regions, as described above, a change in morphology of the cells may be estimated as being ascribed to the cells receiving damage; and a cell protective effect due to a drug may be quantified.

The main types of drug to be subjected to drug screening using cells include drugs which protect cells to reduce cell damage, and drugs which have an effect of controlling cell activation to suppress cell division or accelerate cell growth.

In a process involving cell death due to receiving damage, the shape thereof becomes round and external organelles, such as protrusions, contract. Also, at the stage where cells are completely dead, they become much smaller than normal cells.

The efficacy of a drug that protects cells includes the ratio of the number of dead cells and the number of surviving cells with in response to fixed damage, or the circularity of the cells as a feature which represents the efficacy in more detail.

On the other hand, with a cell that is activated by some effect of a drug, for example, external organelles are observed to grow, and the shape is observed to change from a circle to a complex shape such as an ellipse due to the movement of the cell itself on the culture plate.

The ninth aspect or the tenth aspect described above may further include interpreting deformation of the cell morphology from a circle as being due to motion of the cell; and using the morphological feature as an indicator for measuring activation of the cell by the drug.

In a screening method for nerve cells, when performing analysis on any selected regions in the cell images and classifying these regions as cell bodies, cell protrusions having a tubular structure, and dead cells or air bubbles, which are non-cell regions, on the basis of the parameterized value, information about the classified cell protrusions having a tubular structure may be used as the morphological feature; a total amount of tubular structures may be estimated as correlating with damage that the cells receive; and an effect of a drug may be quantified.

In the ninth aspect or the tenth aspect described above, cell samples formed of a plurality of types may be used.

When using cell samples formed of the plurality of cell types described above, identical evaluations may be performed for a target cell type and other cells types.

A screening apparatus according to an eleventh aspect of the present disclosure includes an illumination unit for irradiating cells with illumination light; an image-information inputting unit for detecting light emitted from the cells to capture image information; an image-information processing unit for parameterizing a morphologicl feature representing a morphological characteristic of each cell, in the image information captured by the image-information inputting unit; an evaluation unit for evaluating the morphological feature parameterized by the image-information processing unit and performing drug screening on the cells; and an output unit for displaying and outputting results of the drug screening by the evaluation unit.

In the eleventh aspect described above, the evaluation unit may use an amount of deviation of a cell outline from a circle or a sphere as the morphological feature.

In the eleventh aspect described above, the evaluation unit may determine the viability of each cell on the basis of the morphological feature.

In the eleventh aspect described above, a plurality of types of cell samples may be used, and identical evaluations may be performed on a target cell type and other cell types.

According to the present invention, it is possible to perform detailed analysis of cell populations. By investigating the correlations of feature changes, it is possible to perform, for example, quantitative measurement and characterization of cellular changes caused by a specific compound and to efficiently compare the effects of a plurality of compounds.

By analyzing the positions of cells on an image, it is possible to predict the influence of inter-cell interactions on these changes, and compared with analysis where the positional relationship of each cell is not considered, it is possible to perform more detailed compound analysis.

The present invention affords an advantage in that it is possible to reduce examiner load and examination error and to accurately count the number of cells.

The present invention affords an advantage in that it is possible to perform drug screening with higher precision by using appropriate morphological features.

Brief Description of Drawings

Fig. 1 is a block diagram showing, in outline, the configuration of a cell-image analysis apparatus according to a first embodiment of the present invention.

Fig. 2 is a flowchart showing the flow of image processing according to the first embodiment of the present invention.

Fig. 3 is a diagram for explaining requirements for setting a plurality of parameters.

Fig. 4 is a scatter diagram for explaining the correlation between parameters.

Fig. 5 is a diagram showing examples of cell shape.

Fig. 6 is a diagram showing the correlation in features of a cell such as that having an anomalous shape.

Fig. 7 is a diagram showing the overall configuration of a cell-image analysis apparatus according to a second embodiment not forming part of the present invention.

Fig. 8 is a perspective view showing an example of a sample plate used in the cell-image analysis apparatus in Fig. 7.

Fig. 9 is a diagram showing an example image of cells in a single well of the sample plate, acquired by the cell-image analysis apparatus in Fig. 7.

Fig. 10 is a diagram showing an example image in which the cells in the same well as in Fig. 9 are acquired after a predetermined time.

Fig. 11 is a diagram showing the example images in Fig. 9 and Fig. 10, together with corresponding example images subjected to binarization processing, by an image-cell analysis method according to a second embodiment of the present invention.

Fig. 12 is a diagram showing an example image of cell nuclei in a single well of the sample plate, acquired with a cell-image analysis method according to a third embodiment not forming part of the present invention.

Fig. 13 is a diagram showing an example image of the cell nuclei in which the same well as in Fig. 12 is acquired after a predetermined time.

Fig. 14 is a diagram for explaining a cell-nucleus identifying method using the cell-image analysis method according to the third embodiment of the present invention.

Fig. 15 is a graph showing the efficacy of a drug, in terms of degree of cell proliferation, using a cell-image analysis method of the present invention.

Fig. 16 is a flowchart of a portion common to the cell-image analysis methods according to the second and third embodiments not forming part of the present invention.

Fig. 17 is a flowchart of the cell-image analysis method according to the second embodiment not forming part of the present invention.

Fig. 18 is a flowchart of the cell-image analysis method according to the third embodiment not forming part of the present invention.

Fig. 19 is a flowchart showing the flow of image analysis processing.

Fig. 20 is a flowchart showing a screening procedure.

Fig. 21 is a diagram for explaining an example feature extraction method in conventional screening.

Fig. 22 is a diagram for explaining a feature extraction method in a screening method according to a fourth embodiment not forming part of the present invention.

Fig. 23 is a diagram for explaining the feature extraction method when performing three-dimensional analysis, in the screening method in Fig. 22.

Fig. 24 is a schematic diagram showing a screening apparatus according to the fourth embodiment not forming part of the present invention.

Fig. 25 is a flowchart for explaining a screening procedure using the screening apparatus in Fig. 24.

Fig. 26 is a diagram for explaining a case where it is necessary to perform three-dimensional analysis in the screening method in Fig. 22.

Best Mode for Carrying Out the Invention

First Embodiment

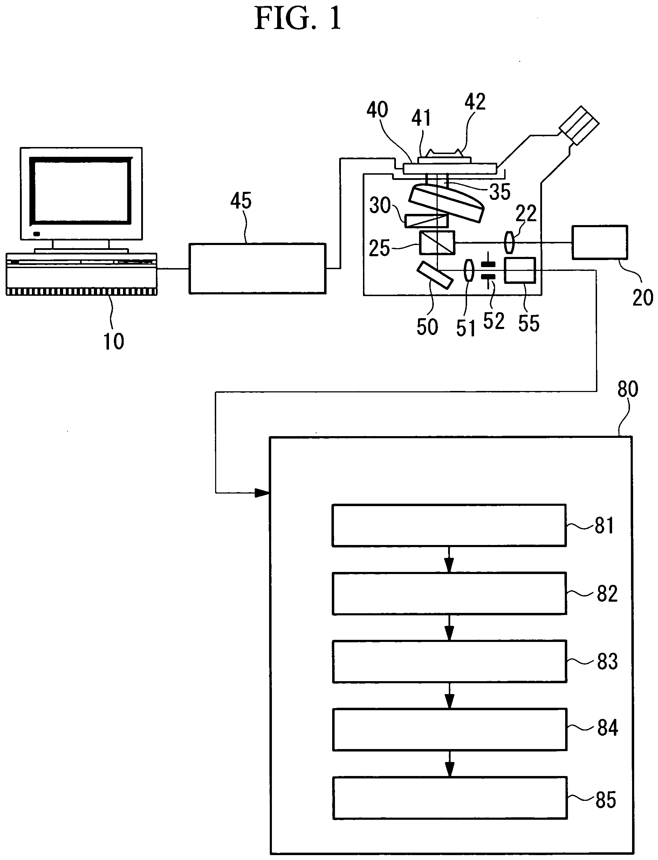

A first embodiment of the present invention will be described with reference to the drawings. The basic configuration for realizing the present invention will be described using Fig. 1. Fig. 1 is a block diagram showing, in outline, the configuration of a cell-image analysis apparatus according to a first embodiment of the present invention, in which a scanning confocal optical microscope is illustrated as an example of the basic configuration of the cell-image analysis apparatus according to this embodiment.

An argon laser with a wavelength of, for example, 488 nm is used as a light source 20. The beam diameter of the laser light emitted from the light source 20 is expanded by a collimator lens 22 to be converted to a collimated beam, and is guided to a dichroic mirror 25. The laser light guided to the dichroic mirror 25 is reflected at the dichroic mirror 25, is incident on an XY scanner 30, and is scanned in the X and Y directions at the scanner 30. The laser light passing via the XY scanner 30 is incident on an objective lens 35 and is focused onto a sample 42 formed of culture cells cultured in a culture solution, which serve as the objects to be measured. The dichroic mirror 25 reflects light with the wavelength of the laser light from the light source 20 and transmits light with wavelengths longer than the wavelength of the laser light from the light source 20. A device in which a pair of galvanometer mirrors oppose each other is normally used as the XY scanner 30.

The sample 42 is tagged with a fluorescent substance and is placed on a microplate 41 mounted on a sample stage 40 of the microscope, which is described in detail later. For example, rhodamine green (RhG) is used as the fluorescent substance. Fluorescence emitted from the sample 42 passes again through the objective lens 35, via the XY scanner 30, passes through the dichroic mirror 25, is reflected at a mirror 50, and reaches a lens 51. After the fluorescence is converged by the lens 51 to be focused at an opening of a pinhole 52, it is received at a light detector 55. A fluorescence signal received at the light detector 55 is converted to a photocurrent signal, is input to an image processing unit 80, where computations such as signal processing and image analysis, which are described in detail later, are performed, and a fluorescence image of the sample is extracted on a monitor. A standard photomultiplier tube (PMT) is used as the light detector 55. Alternatively, a high-sensitivity semiconductor detector such as an avalanche photodiode (APD) may be used.

Although a method of scanning the sample and extracting an image of the sample is described using Fig. 1, it is not limited thereto. It is also possible to irradiate the sample with uniform light and, using fluorescence emitted from the sample, capture an image of the sample using a CCD (Charge Couple Device) camera, a CMOS (Complementary Metal Oxide Semiconductor) image sensor, or the like. In such a case, a mechanism for optically scanning the sample is not necessary.

The microplate 41, which has 96 holes and whose bottom surface is transparent, for example, is used as a container for holding the sample 42. Immersion liquid fills the space between a tip of the objective lens 35 and the microplate 41. The microplate 41 is supported on the sample stage 40. Two stepping motors (not shown) are connected to the sample stage 40 so as to enable the sample stage 40 to move in two orthogonal direction, and each stepping motor is connected to a sample stage controller 45. The sample stage controller 45, which is connected to a computer 10, holds the sample stage 40 and moves the sample stage 40 in the X-direction and the Y-direction by driving the stepping motors based on commands from the computer 10 to position the sample 42 at an appropriate location.

By moving the objective lens 35 in the Z-direction, it is possible to move the focus position of the laser light up and down in the Z-axis direction.

The fluorescent substance is not limited to rhodamine green; it is also possible to use, for example, TMR (tetramehylrhodamine), Cy five (Cy5), FITC (fluoresceinisothiocyanate), TOTO1, Acridine-Orange, or Texas-Red, so long as it is compatible with the wavelength of the light source.

The output image from the detector 55 is input to the image processing unit 80, where various types of image processing are performed. The image processing unit 80 includes a cell-center extraction processing unit 81, a center-point examination processing unit 82, a cell-boundary extraction processing unit 83, a cell-feature extraction processing unit 84, and a cell statistics processing unit 85. First, based on the output image from the camera 55, the cell-center extraction processing unit 81 detects and extracts points forming the centers of the individual cells in each image. By comparing the points extracted in the cell-center extraction processing unit 81 for a plurality of images, the center-point examination processing unit 82 extracts the cell centers, excluding unsuitable detection results (that is, areas regarded as detection errors), as the centers of the cells. The cell-boundary extraction processing unit 83 extracts the most suitable boundaries enclosing the cell centers extracted in the center-point examination processing unit 82. The cell-feature extraction processing unit 84 extracts morphological features of individual cells based on the results of the cell-boundary extraction processing unit 83. The cell statistics processing unit 85 analyzes the features extracted in the cell-feature extraction processing unit 84 and extracts the number of cells included in the image.

For the hardware constituting the image processing unit 80, it is possible to use a general-purpose processing device operated by a computer program, for example, a general-purpose computer or a personal computer. Alternatively, the image processing unit 80 may be part of the computer 10. In such a case, the processing device described above has a program installed therein for making it function as the image processing unit 80, in other words a program for realizing the individual functions of the cell-center extraction processing unit 81, the center-point examination processing unit 82, the cell-boundary extraction processing unit 83, the cell-feature extraction processing unit 84, and the cell statistics processing unit 85, to constitute a cell-image processing device.

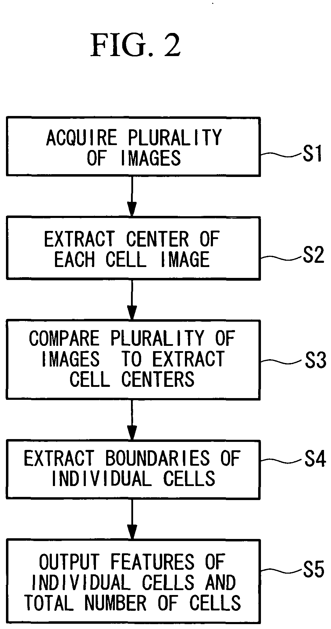

The operation of the cell-image analysis apparatus according to the first embodiment of the present invention, configured as described above, will be described with reference to Fig. 2. Fig. 2 is a flowchart showing the flow of image processing according to the first embodiment of the present invention. The description given below will follow the processing flow.

In general, acquisition of cell images is frequently carried out by magnifying, with a microscope or the like, light colorized by staining protein in the cells and the nuclei of the cells, or by using a fluorescent protein (such staining and so on is called "sample manipulation").

This image acquisition is performed by acquiring multiple images in the following procedure (step S1).

(1) In cell sample preparation, cells to be observed under various conditions are prepared.

(2) Sample manipulation is carried out on the prepared cell samples. Here, for samples in which there are plural types of cells, it is possible to manipulate them using a staining method so that only a specific type of cell emits light. Using this property, cells to be observed are subjected to characteristic staining or light-emission manipulation appropriate to the cells.

(3) After sample manipulation, the sample 42 is mounted on the stage 40 of the cell-image analysis apparatus and is fixed in position.

(4) An image of the sample 42 mounted on the stage 40 (in other words, a "cell image") is extracted on the monitor of the computer 10. In this case, with the sample remaining fixed in position, the image acquisition conditions are changed, such as changing a filter, a required number of times, and image acquisition is performed each time the image acquisition conditions are changed.

(5) Using the microplate 41, the cell position is changed, and similarly to (4), a plurality of cell images are acquired while changing the image acquisition conditions.

When cell images are acquired in the way described above, the cells in the image are recognized for each image, and the centers thereof are extracted in the cell-center extraction processing unit 81. In this cell recognition, binarization of the images is performed using, for example, a suitable luminance value, and regions including the cells are estimated from the boundaries in the binarized images. By doing so, the cells are recognized to identify the position of each cell, and the centers thereof are extracted (step S2).

Because there is a possibility of erroneously extracting the cell centers in step S2, by comparing a plurality of cell images acquired at the same position, cell center positions that are determined to be correct are extracted in the center-point examination processing unit 82 (step S3).

The boundaries of cells corresponding to the cell centers extracted in step S3 are extracted in the cell-boundary extraction processing unit 83 (step S4). In the boundary extraction in this step, the image data should be binarized, similarly to step S2 for example, and boundaries should be extracted so that they contain the cell centers extracted in step S3. With this step, it is possible to recognize cells with high precision.

Once the cell recognition (identification of each cell) is completed, extraction of the features by the cell-feature extraction processing unit 84 and extraction of the total number of cells by the cell statistics processing unit 85 are performed, and the features and the total number are output (step S5). Based on the cell boundary information extracted in the cell-boundary extraction processing unit 83 a plurality of cell features are extracted, for example, the size of a cell, the maximum radial position, and the length of the perimeter, or the cell brightness, the systematic error in the cell brightness, and so forth.

By extracting this features and performing a comparison test, it is possible to evaluate the efficacy of a drug. For example, it is possible to evaluate the efficacy of the drug by dividing the cells into cells that are administered a compound and cells that are not administered a compound, culturing these cells under fixed conditions, and comparing them. More specifically:

> (1) The cells are divided into two cell groups, and the compound is administered to one group. The other cell group is not administered anything.
> (2) The two cell groups are cultured for a fixed period of time.
> (3) With the number of cells as a parameter, image acquisition and analysis of the cell groups is performed with the method described above, and the numbers of cells in the two groups are compared.

When, for example, the culturing period is increased in the evaluation test as described above, a certain amount of cell death is expected. However, if the number of cells surviving in the cell group administered the compound is clearly higher than the number of cells not administered the compound, it can be stated that the compound has some protective effect on the cells.

For example, when taking the length of a protrusion of each cell as a parameter, the protrusion length is measured after administering the compound, and the elongation of the protrusion is compared for each cell. At this time, if the elongation of the protrusion is significantly larger in the cells administered the compound, it is possible to conclude that the compound has an effect causing the cell protrusion to extend.

Thus, in this embodiment, with various types of analysis, it is possible to extract cell features and to evaluate the efficacy of the drug. More specifically, in this embodiment, a statistical analysis method for extracting features and so forth will be described below.

Taking the distribution of cell size as an example, the ideal statistical distribution is given by a normal distribution, a Poisson distribution, or a binomial distribution. The characteristics of the distribution in this case can be represented by several statistical quantities, such as the average value, the variance, and so forth.

When screening a compound using cells, the distribution of a single parameter is not limited to a well-known distribution such as those described above. Therefore, in the embodiment of the present invention, more detailed statistical analysis of the cell parameters is performed by detecting statistical quantities such as those described above and, in addition, by detecting the peak position.

In particular, one example of a method for detecting the peak position and predicting the efficacy of the compound from the characteristics thereof is given below.

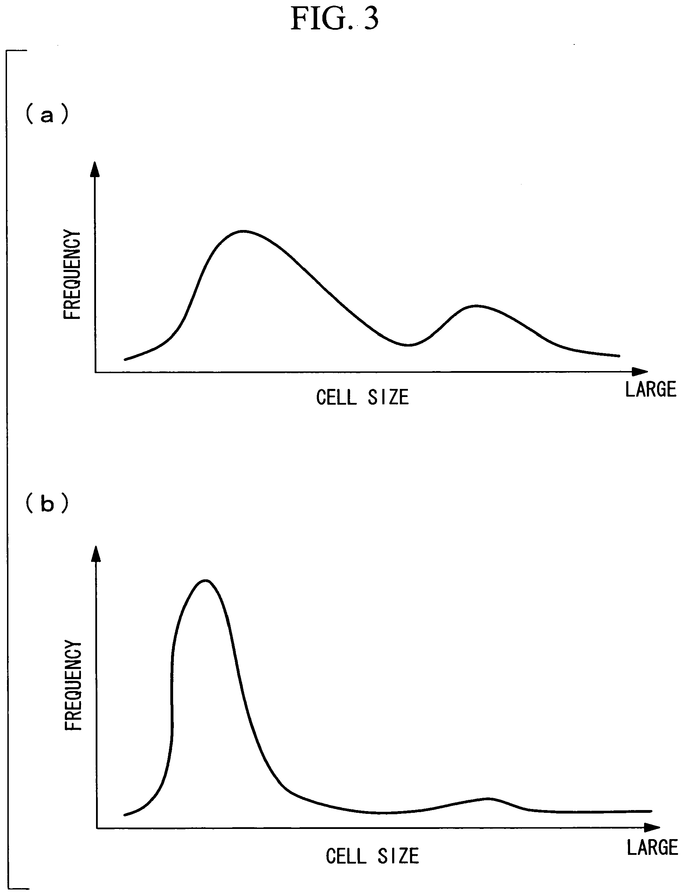

A distribution graph is created with the parameter on the horizontal axis and the frequency on the vertical axis, as in Fig. 3. In Fig. 3, the frequency of the distribution is normalized. (a) shows the distribution in the case where the compound is not administered, and (b) shows the distribution in the case where the compound is administered. In this case, if the cell size is taken as the parameter, the average value thereof is close to the peak position at smaller sizes, and it is not possible to sufficiently represent the difference with the average value only. In other words, it is not possible to sufficiently distinguish the characteristics of the distributions in (a) and (b) by using only a simple average. In addition, particularly in (a), it is not possible to represent two peaks using only the average value.

However, as is clear from the distribution curve in Fig. 3, because there is a large peak at smaller sizes in (b), the compound has an effect of increasing the ratio of cells having a smaller size value. In other words, this can be interpreted as the compound acting on large cells, making them shrink. By setting parameters for various features (hereinafter referred to as "feature parameters"; cases where they are called simply "parameters" also indicate these feature parameters, unless otherwise stated), interpretations that agree with the characteristics of each compound are thus possible.

Next, when a plurality of feature parameters are set, a method for evaluating the effectiveness of the compound by verifying the correlatiions of the plurality of feature parameters will be described. By verifying the correlations of the plurality of feature parameters, the following is found. That is, it is possible to verify the measurement accuracy of each parameter and to select the most appropriate parameter for the experimental system; as a result, it is possible to select or design the most appropriate parameter for cell evaluation.

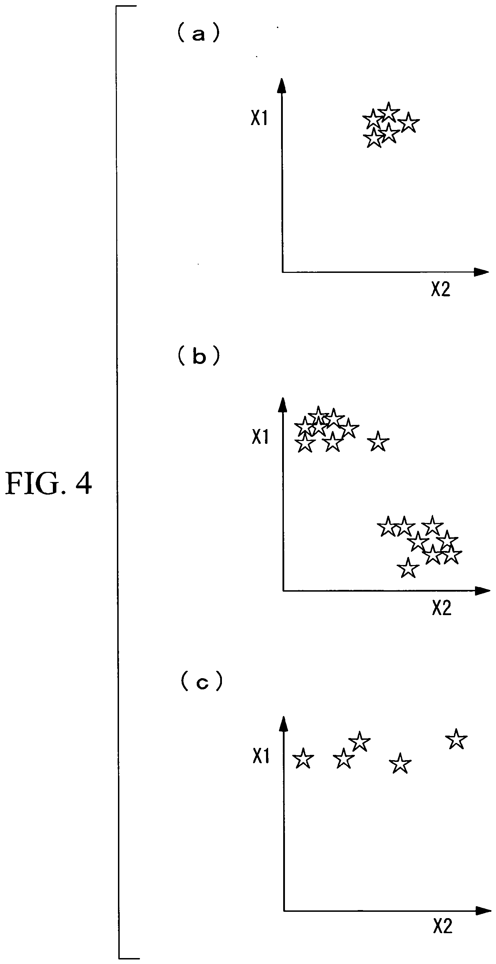

In correlations of parameters, normally, it is known that there are positive correlations and negative correlations. A positive relationship is shown, for example, in Fig. 4, which will be described in detail later. A relationship such that the radius increases as the size increases is called a positive correlation. Conversely, a relationship such that the radius decreases as the size increases is called a negative correlation.

Correlations will be described in more detail with reference to Fig. 4. Fig. 4 is a scatter diagram for explaining the correlations. In Fig. 4, the vertical axis is defined as parameter $x_1$ and the horizontal axis is defined as parameter $x_2$. For example, the radius and the size may be used as parameters, but they are not limited thereto. Any indicators may be defined as the parameters so long as they represent features of the cell.

In Fig. 4, first, when comparing the two graphs in (a) in Fig. 4 and (c) in Fig. 4, generally (a) in Fig. 4 is determined to have a strong correlation, and (c) in Fig. 4 is determined to have a weak correlation. In the distribution determined as having a weak correlation in (c) in Fig. 4, the scatter of the parameter x1 is small, and is distributed around substantially constant values. On the other hand, the parameter x2 is not distributed around a specific value, but is distributed almost randomly. Therefore, (c) in Fig. 4 is considered to be a distribution in which the parameter x2 is not correlated with the parameter x1, and it is thus determined the these two parameters are not correlated. In (a) in Fig. 4, since the two parameters x1 and x2 are distributed around constant values (for example, the average value), in this case, they are considered to have a positive correlation, for example.

A distribution having a negative correlation, such as that in (b) in Fig. 4, can also be assumed, and this kind of distribution is also determined to have a strong correlation. As shown in (b) in Fig. 4, the parameter x2 increases as the parameter x1 decreases.

From the examples described above, the strength of the correlation is given as follows. In the case of a strong correlation, the distribution on the vertical axis is reduced when the value on the horizontal axis is fixed. In other words, a characteristic whereby the vetical axis shows a wide distribution around the average value at that value is observed.

For example, in Fig. 4, it is assumed that the parameters x1 and x2 take discrete values (i < 1, 2,...). In this case, first, the parameter x2 is fixed, the distribution of the parameter x1 corresponding to the value of parameter x2 is examined, the average thereof is taken, and a difference from this average is obtained. Then, by performing the same operation by changing the value of the parameter x2, it is possible to estimate the correlation between the two parameters.

The correlation is given by equation (1):

$$(1/N0)\, \mathtt{Sum\_\{i<N\}}\, (1/\mathtt{Misum\_\{j<Mi\}}|y(i,j)\cdot \mathtt{avr}(y(i)|), \quad (1)$$

where N0 is the total number of data points, i is the value on the horizontal axis, j is the value on the vertical axis, $y(i, j)$ are the values distributed at each fixed i, and $\mathrm{avr}(y(i))$ is the average of each i.

It is thus possible to quantify the correlation between the two parameters with the method described above. In the case of multiple groups of parameters exceeding two, by quantifying the level of correlation for each pair, it is possible to calculate the correlations of all parameters.

Next, narrowing down of parameters will be described.

Parameters generally represent features of the cells. Features of the cells include the size and radius, as described above, as well as the perimeter length of the cell boundary, and so on. Evaluation of a system, such as an experimental one (hereinafter simply referred to as "experimental system"), is carried out using the values of these features.

For example, consider the case of evaluating a drug having an effect of protecting cells from some stimulus. The experimental system here is formed of cells, an apparatus for culturing them for a fixed period of time, a stimulus which is applied to the cells, and so on. Evaluation of a drug using this system is conducted based on, for example, the average number of cells present in a fixed region after applying a stimulus, post-administration, and waiting for a fixed period of time.

In this embodiment, only the number of cells is selected as the cell feature; other features such as the size and so forth of each cell are not used directly for evaluation. Thus, evaluation based on features is not performed by selecting many parameters, but is frequently performed by selecting (in other words, narrowing down to) only a few parameters.

One reason for performing evaluation by narrowing down the parameters is that there are many instances where accurate measurement or estimation of the feature parameters is itself difficult, it is generally troublesome to handle multiple parameters, and so forth.

Therefore, the first embodiment of the present invention is characterized in that representative parameters are narrowed down and selected from a plurality of parameters, and the system is designed so that the selected parameters match the experimental system. In this case, it is important to consider the effectiveness of the parameters. Effectiveness of a parameter means that the correlation between the effect of the parameter and the drug efficacy is strong. For example, as described above, when considering the correlation between the cell protective effect of the drug and the number of cells, generally the correlation is strong. In other words, after the cells receive a stimulus, if many more of them survive than in the case where the drug is not administered, the administered drug can be determined to be effective. Thus, it is presumed that it is possible to determine efficacy based on the number of cells, and therefore, defining the number of cells as a parameter is effective. The effectiveness of a parameter is not determined just based on specific knowledge, such as "the relationship between the cell protective effect of the drug and the number of cells" described above. For example, an evaluation function given by a numerical expression can also be a valid parameter. Also, a parameter for comparing correlation values is another measurement parameter.

Consider this from the viewpoint of a data space. It is assumed that there are n types of cell features (in other words, there are n parameters), and the parameter values are defined as x1, x2, ..., xn. Here, the parameters are represented by (x1, x2, ..., xn), which are points in an n-dimensional data space.

Here, for example, x1 is the average number of cells present, and x2 is the average size or the like of the cells. The efficacy is thus assumed to be converted to numerical values, such as al=100, a2=70, etc.

The "designed parameters" for representing the efficacy can be written as a function defined in the n-dimensional data space, such as F(x1, x2, ..., xn).

The correlation between this function and the efficacy is defined, for example, by equation (2):

$$(1/N)\,sum\_\{i<N\}|ai - Fn(di)|(di=(x1(i), x2(i), ..., xn(i))).$$

$$(2)$$

Here, ai is a correct measurement value of the efficacy (reference data that is measured in advance), and Fn(di) is the result of the function Fn in which an i-th data value di is substituted. The average of the differences between the measured values of the efficacy and the predicted values which are calculated is taken in equation (2); therefore, if the correlation is high, this value is small.

Next, consider the case of multiple functions F1, F2, ..., Fm. Here, Fi (i = 1, 2, ..., m) are functions having different definitions that can be expressed with single variables, for example, F1 = x1 and F2 = x2, or that can also be expressed with an arbitrary equation, such as, F3 = x2 - x3. By calculating the above-mentioned correlation with respect to the function Fi, the one whose function value (error value) is lowest (one with a high correlation) is defined as the most "effective function". In this specification, this "most effective function" is defined as the "most effective parameter". For example, considering the case where a function such as F1 = x1, F2 = x2, etc. is represented with a single parameter, this representation (effective parameter) is understood to be an appropriate representation.

In particular, in the actual calculation, it is preferable that the number of variables be small. The reason for this is to perform measurement and calculations in a short period of time. It is also beneficial in that it is easy to intuitively understand the relationship between variables and efficacy in a simple manner.

As described above, in the first embodiment of the present invention, correlation of the parameters is taken, and modeling is performed with a function in which parameters are narrowed down according to the experimental system or in which the optimum parameters are used. Then, in particular, the effectiveness of the parameters is defined as a "function producing the smallest error value in the result", as in equation (2). As concrete ways of obtaining the function, optimum parameters are given by (1) evaluating the measurement accuracy and reliability of the parameters, and (2) representing variations, and so on.

As an example of analysis using parameters and the correlation thereof, evaluation (verification) of measurement accuracy will be described. As a simple example, a method of investigating the correlation of the parameters cell size and cell peripheral length and verifying whether the cell size is correctly measured will be described.

It is already known that the cell size, given as the maximum width of the cell boundary, and cell perimeter length are given by a simple relationship when the cell shape is spherical or ellipsoidal. Therefore, when, for example, the cell and its boundary are correctly detected, the two parameters are expected to have a simple relationship according to this relational expression.

The relationship between the cell size and the cell perimeter length in the distribution in Fig. 4 is expected to be a distribution close to a proportional relationship, in other words, a linear distribution on the scatter diagram.

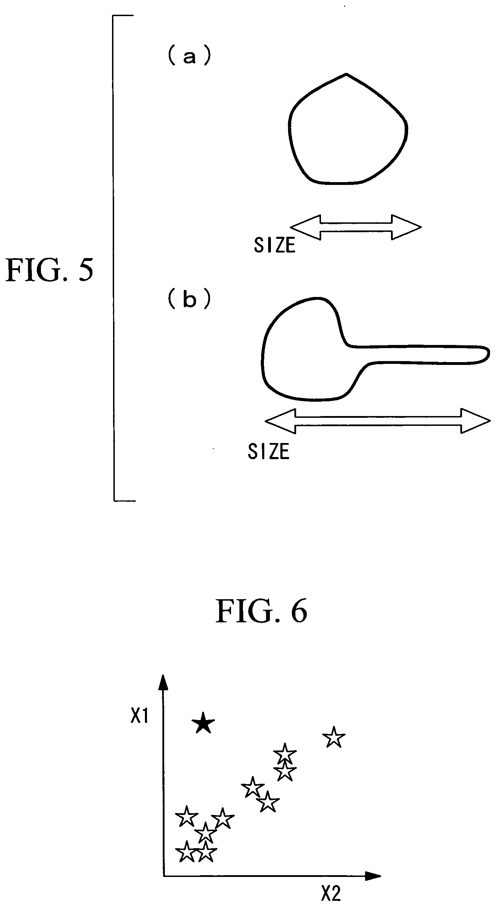

However, when, for example, an object other than the original object to be detected is included in the image, it is conceivable that, for example, other than an object with a shape like that in (a) in Fig. 5, an object with a shape like that in (b) in Fig. 5 is detected. In such a case, the relationship between the size and perimeter length deviates drastically from a simple relational expression such as that originally predicted.

Here, when the peripheral length is on the vertical axis and the radius is on the horizontal axis, the distribution related to these parameters is predicted to be as shown in Fig. 6. However, a cell having an anomalous shape, such as that shown in (b) in Fig. 5, is observed at the position of the black star in Fig. 6.

When the average values on the vertical axis and the horizontal axis and the average values of their variation are calculated, for example, such an anomalous shape can be detected as a point that is shifted by a large amount from the average. This variation can be quantified with an expression similar to equation (1). In the case of a group of parameters whose correlations can be explicitly predicted in advance in this way, it is possible to evaluate the experimental system or measurement method by taking the actual correlations of the parameters.

Thus, by verifying the correlation with respect to the measured value of each parameter, it is possible to estimate the precision of the measurement itself. In addition, when verifying the correlation of, for example, cell size and cell perimeter length, in this way, it is possible to exclude incorrect cell detection data; therefore, it is possible to properly correct all measurement results. In other words, in the example shown in Fig. 6, it is possible to exclude the measurement point indicated by the black star based on the variation from the average.

Although the precision of the measurement itself has been described above, similarly for changes due to the conditions, by verifying the correlation of each parameter, it is possible to select parameters which most effectively represent the changes due to the experimental conditions and so forth.

In general, analysis of cell images does not really have high precision. In particular, when the experimental system or experimental conditions change, the errors become extremely large. Therefore, it is necessary to set multiple evaluation parameters even for a single evaluation system. In this case, when evaluating the efficacy of a compound, a system for evaluating, for example, the effect of protrusion extension is considered. Here, detection of the protrusion is not always perfect, and therefore, detection of multiple parameters, such as the maximum diameter, the peripheral length, and the surface area of each cell, is performed.

When evaluating this protrusion elongation, it is necessary to correctly recognize the cell, correctly recognize the cell boundary, correctly recognize the cell protrusion position, and correctly recognize the length of this protrusion. In general, however, it is difficult to automatically perform all of these recognition processes in software.

Thus, as in the first embodiment of the present invention described above, the correlation between a plurality of types of detected data and the detected elongation of the protrusion should be taken, and weighting should be applied according to that correlation. Alternatively, the parameter with the highest correlation may be taken as a reference parameter and used as basic data together with changes in the reference parameter.

With the analysis described above, parameter design becomes possible, such as selecting the most suitable parameter for evaluating the efficacy of a drug on a cell, changing the weighting, and so forth. Therefore, it is possible to perform cell analysis effectively.

An example of analysis using position information of cells will be described. A case where an experiment is conducted with the aim of searching for a compound having an effect causing long-term survival of cells is illustrated as an example, and the effect of the compound is measured by counting the number of cells.

A case where the ratio of cell numbers is high for a region with a high cell density or for multiple cells in contact can also be interpreted as producing conditions whereby some kind of effect takes place between cells, which interacts with the compound, and more cells survive as a result.

Conversely, it is assumed that reactions that are not influenced by interactions between cells are to be investigated. In this case, it is possible to adopt a method in which only the features of free-swimming cells or cells whose nearest cells are at a fixed distance or greater are extracted, and the results thereof are evaluated.

With such a method, it is possible to create a particularly effective evaluation system if, for example, the work required for 50% cell isolation is drastically less than the work required for 100% isolation.

With the first embodiment of the present invention described above, because the processing is automated, it is possible to perform screening efficiently. In addition, because parameter selection is efficient, more effective screening results are expected. It is also possible to perform accurate measurement of the effect of a drug.

The present invention is not limited to the structures of the above-described embodiments. In actual implementation, various modifications are possible so long as they do not depart from the scope of the invention. Moreover, inventions of various levels are included in the structures of the above-described embodiments, and various inventions can be derived by appropriate combinations of the multiple disclosed elements.

For example, even if some element is removed from all elements shown in the embodiments described above, the problems to be solved by the invention section can still be solved, and if advantages of the invention can be achieved, the configuration in which this element is removed can be derived as the invention.

Second Embodiment

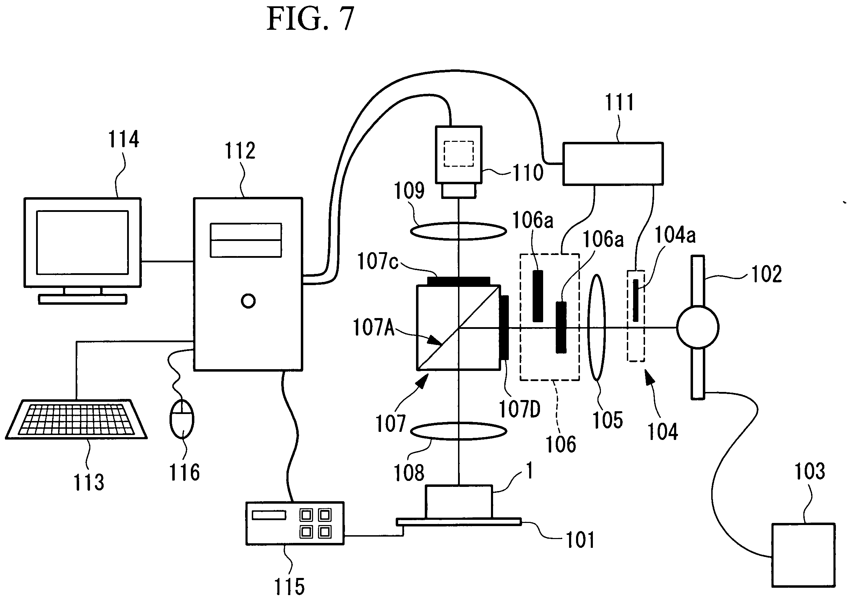

A second embodiment not forming part of the present invention will be described below with reference to Figs. 7 to 11, and 15 to 17.

Fig. 7 shows the configuration of a cell-image analysis apparatus according to the second embodiment not forming part of the present invention.

As shown in Fig. 7, the cell-image analysis apparatus according to this embodiment includes a stage 101 for mounting a sample plate 1; an excitation light source 102; a power supply unit 103 connected to the excitation light source 102; a shutter unit 104 for turning the light from the excitation light source 102 on and off; a lens 105 for converting the light to collimated light; a filter unit 106 for selecting the wavelength of excitation light to be made incident on the sample; a mirror set 107 for reflecting the excitation light to make it incident on the sample and for transmitting fluorescence; an objective lens 108 for focusing the excitation light onto a sample; an image-forming lens 109 for focusing the fluorescence collected by the objective lens 108 and transmitted through the mirror set 107; a CCD camera (image-information input unit) 110 for acquiring the imaged fluorescence; a universal control box 111 for controlling the shutter unit 104, the filter unit 106, and the mirror set 107; a stage controller 115 for controlling the stage 101; and a computer (luminance-value calculating unit, comparison calculating unit, counting unit) 112 for processing the fluorescence acquired by the CCD camera 110 and for controlling the universal control box 111 and the stage controller 115.

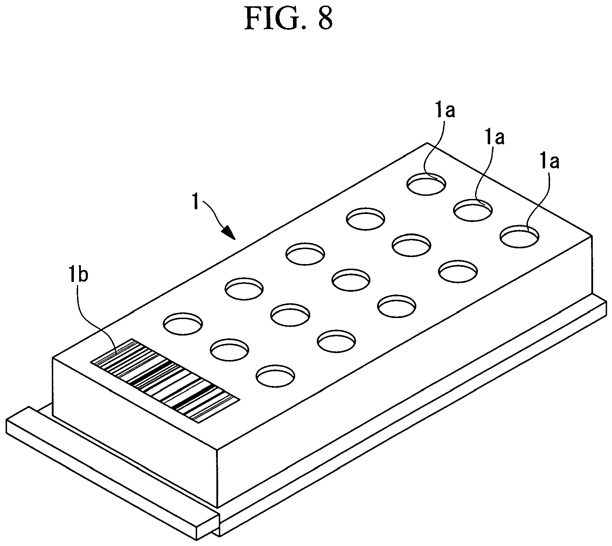

As shown in Fig. 8, a plate in which fifteen wells 1a with, for example, a diameter of 20 mm and a depth of 15 mm are arranged in three rows by five columns is used as the sample plate 1. Cells stained with a fluorescent dye, which serve as the objects to be examined, are disseminated inside each well 1a. The fluorescent dye used in this embodiment is, for example, a dye that can emit fluorescence while the cells are kept alive, such as GFP, and stains the cytoplasm. A barcode 1b is affixed to the sample plate 1. By reading the barcode 1b with a barcode reader that is not shown in the drawing, it is looked up in a database to ensure compliance between the sample plate and the data in the database.

The stage 101, on which the sample plate 1 is mounted, can be two-dimensionally driven with motors in the horizontal directions to position the sample plate 1 at an arbitrary position.

A mercury light source or the like is used in the excitation light source 102.

The shutter unit 104 is positioned in the light path of the excitation light emitted from the excitation light source 102 and has a built-in shutter plate 104a which can be opened and closed.

The filter unit 106 includes two excitation filters 106a and can radiate two kinds of excitation light with different wavelengths towards the sample.

The mirror set 107 is formed of a dichroic mirror 107A for reflecting the excitation light and transmitting the fluorescence, as well as an excitation filter 107D and an absorption filter 107C which are attached thereto.

The mirror set 107 is held in a mirror holder, which is not shown in the drawing, and can be arbitrarily replaced in the light path using a motorized unit, which is not shown in the drawing, according to the required excitation wavelength. The objective lens 108 and the sample plate 1 are disposed in the reflected light path of this mirror set 107. The image-forming lens 109 and the CCD camera 110 are disposed in the transmission light path of the mirror set 107.

The CCD camera 110 is, for example, a 12-bit 1-megapixel camera with $1000 \times 1000$ pixels.

A keyboard 113, a monitor 114, and a mouse 116 are connected to the computer 112.

A cell-image analysis method using the cell-image analysis apparatus according to this embodiment, having the above configuration, will be described in detail below using, as an example, screening of an anti-cancer drug.

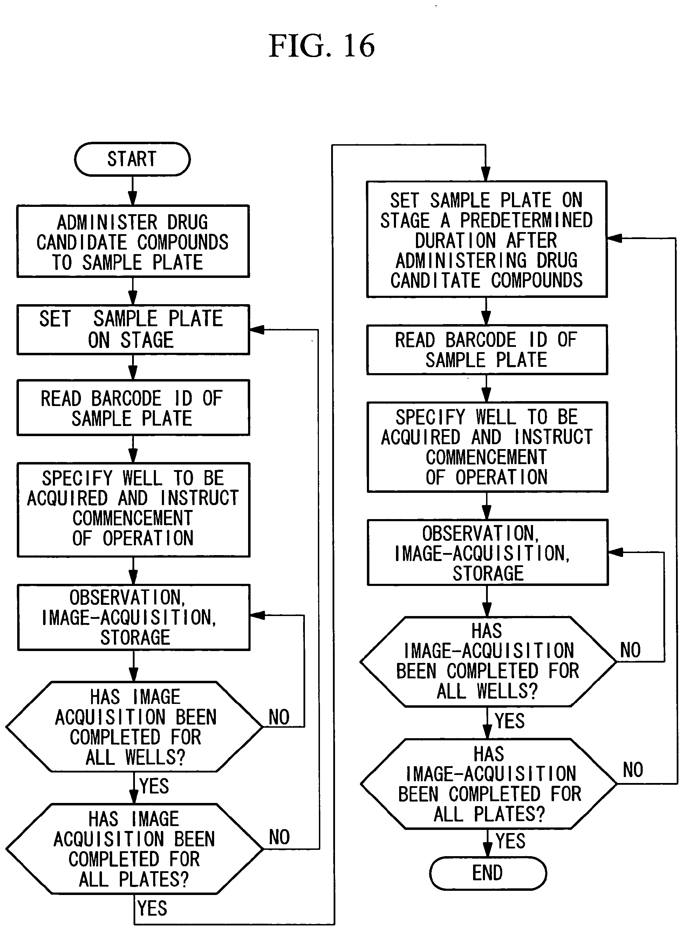

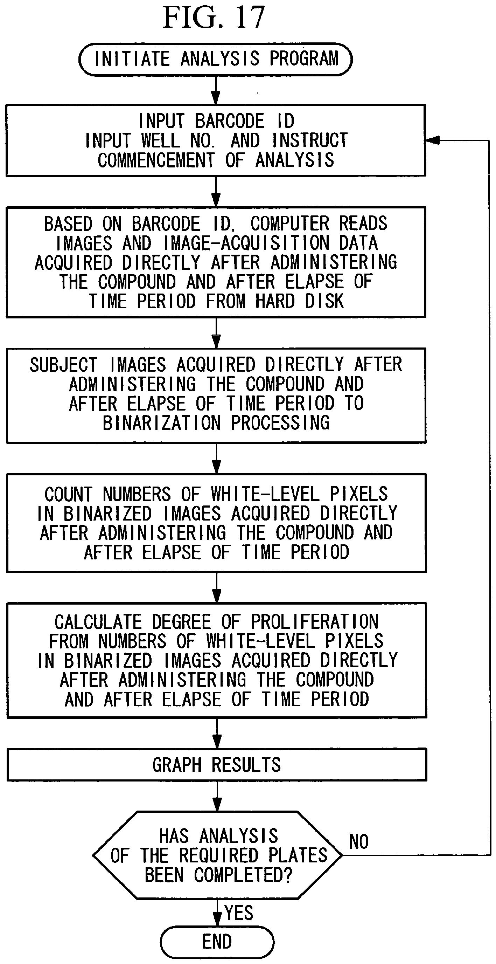

Flowcharts of the cell-image analysis method according to this embodiment are shown in Figs. 16 and 17.

First, cells are inoculated in the first row of wells 1a in the sample plate 1 without administering a drug candidate compound, and cells are inoculated in the second row and are administered a compound A which is a drug candidate. In addition, cells are inoculated in the third row and are administered a compound B which is a drug candidate.

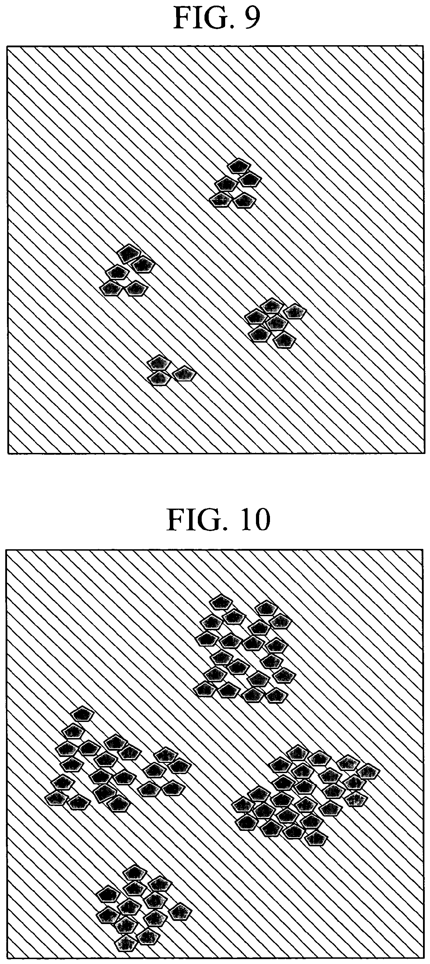

Then, the sample plate 1 is set on the stage 101, a control program that is not shown in the drawing is started up on the computer 112 to read the barcode (ID:10001) on the sample plate 1, and observation/image-acquisition is started. Thus, an image of the cells, shown in Fig. 9, is captured. This is repeated for the required number of sample plates 1.

Then, after two hours, the sample plate 1 is again set on the stage 101, the barcode (ID:10001) of the sample plate 1 is read, the control program (not shown) is executed on the computer 112, and observation/image-acquisition is started. Thus, an image of the cells, shown in Fig. 10, is captured. This is repeated for the required number of sample plates 1.

Once image-acquisition is completed, the examiner proceeds to verification of the acquired cell image. The examiner executes a cell-image analysis program on the computer 112. Then, the examiner inputs the barcode ID of the sample plate 1 to the cell-image analysis program and inputs a well number being examined. It is assumed that all wells are specified at this stage.

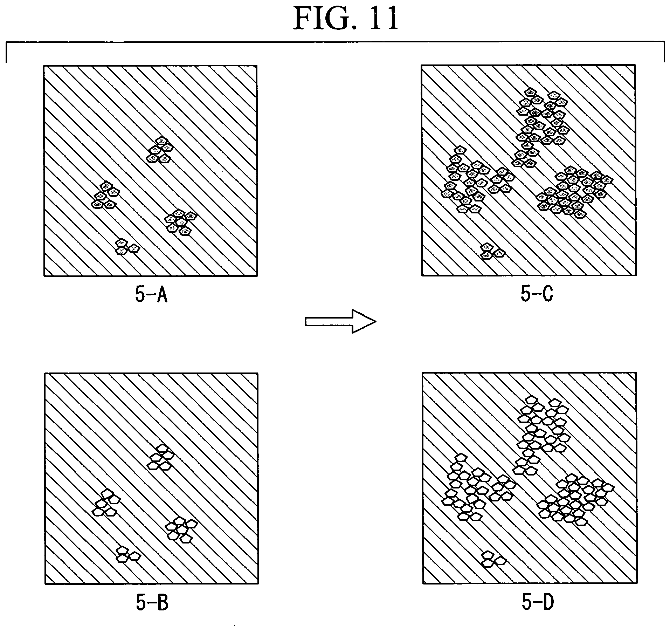

Then, upon instructing commencement of analysis, the cell-image analysis program loads two sets of fluorescence image data at different image-acquisition times, which are already stored in a hard disk of the computer 112, into a memory (not shown) from the barcode ID and the well number. Taking the rightmost well in the first row of the barcode (ID:10001) as an example, the corresponding fluorescence image data loaded in the memory is as shown in 5-A and 5-C in Fig. 11.

The cell-image analysis program binarizes the image in 5-A in Fig. 11 to 5-B and binarizes the image in 5-C to 5-D. The binarization level at this time is the same value for both 5-B and 5-D (800 in decimal). Then, the numbers of pixels at the white level (luminance level of 4095 in decimal) in the images in 5-B and 5-D are counted.

As a result, for example, when the number of white-level pixels in image 5-B is 19,000 and the number of white-level pixels in image 5-D is 74,000, the cell-image analysis program compares them to give a degree of proliferation of the cells in well number 1 of (74000/19000) = 3.89, and displays it. This is repeated the required number of times to display the degree of proliferation of the cells in each well.

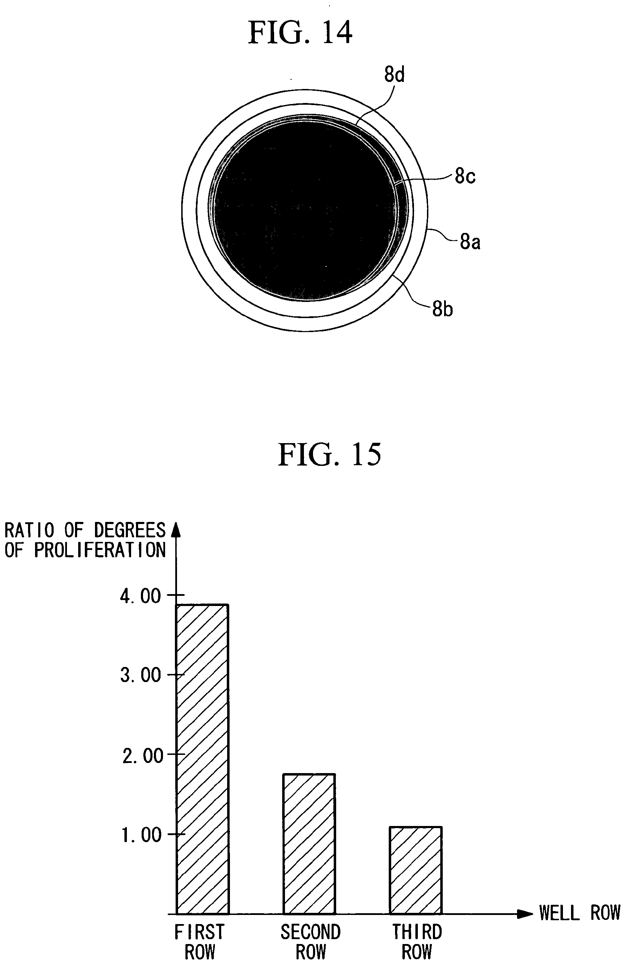

The examiner checks, for example, the degree of proliferation of the cells in the first, second, and third rows of the rightmost wells on the sample plate 1. For example, as shown in Fig. 15, if the first row indicates 3.89 , the second row indicates 1.85, and the third row indicates 1.12, it is found that the effect of the compound B, serving as the drug candidate, in suppressing cell proliferation is the maximum.

By performing this operation for all wells, finally averaging the data in each row, and comparing the degrees of proliferation by row, it is possible to evaluate the efficacy of the compounds serving as the drug candidate.

By examining the degree of proliferation of cancer cells using this principle, it is possible to perform cancer pathology.

Third Embodiment

Next, a third embodiment not forming part of the present invention will be described below with reference to the drawings.

In the description of these embodiments, parts having the same configuration as those in the second embodiment described above are assigned the same reference numerals, and a description thereof is omitted.

The sample plate 1 used in this embodiment is the same as that used in the second embodiment; it has 15 wells 1a, with a diameter of 20 mm and a depth of 15 mm, arranged in three rows by five columns. Cells stained with fluorescent dye, serving as the objects to be examined, are inoculated in each of these wells 1a. The fluorescent dye used in this embodiment is a dye that can emit fluorescence while the cells are kept alive, for example, GFP, and dyes the cell nucleus.

Screening of an anti-cancer drug is described as an example.

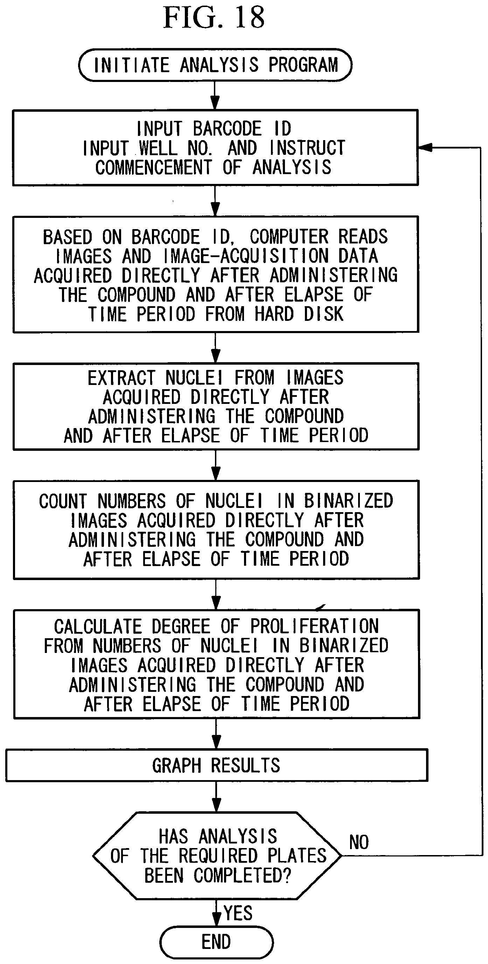

Flowcharts of the cell-image analysis method according to this embodiment are shown in Figs. 16 and 18.

The examiner inoculates cells into the first row of wells 1a of the sample plate 1 without administering a compound which is a drug candidate, and then inoculates cells into the second row and administers a compound A which is a drug candidate. In addition, he or she inoculates cells into the third row and administers a compound B which is a drug candidate.

Once sample preparation has been completed, the sample plate 1 is set on the stage 101, a control program which is not shown in the drawing is started up on the computer 112 (luminance-value calculating unit, comparison calculating unit, counting unit) to read a barcode (ID:00001) on the sample plate 1, and observation/image-acquisition is started. Based on this start instruction, the universal controller box 111 and the stage controller 115 start operating, and a fluorescence image of the cells in a specified well is acquired.

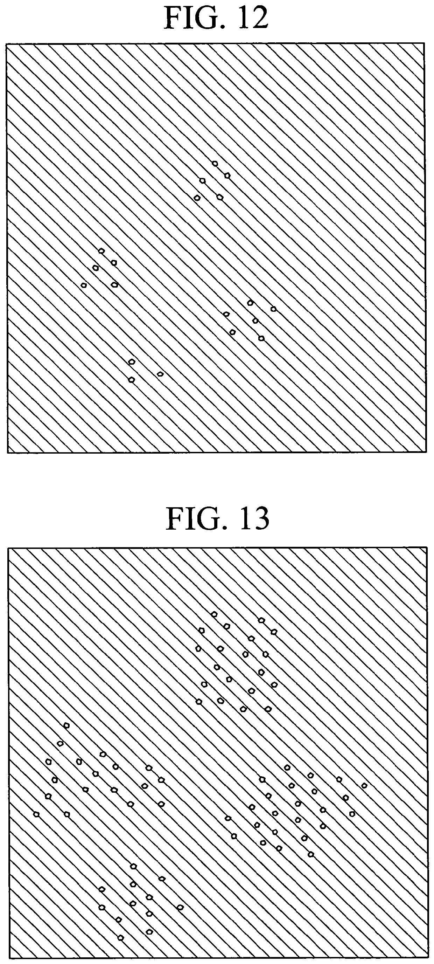

The well being acquired can be arbitrarily specified in the control program. In this embodiment, it is set so that all fifteen wells are acquired. Fig. 12 is a fluorescence image of the rightmost well in the first row, with barcode (ID:00001), acquired immediately after commencing examination. It is clear that the nuclei of the cells emit light due to the fluorescent dye. The cell nuclei are all substantially circular, and the nuclei are of such a size that they are also contained in a fixed boundary. The cells form a number of close-packed groups (four in the case of this well). This is repeated 15 times to acquire fluorescence images of the cells in all wells.

The acquired image is stored in the hard disk of the computer 112 together with the barcode ID and the well No. of the sample plate 1, and data about the image-acquisition time and the exposure time. Instead of the hard disk, it is also possible to use a semiconductor memory or the like which can be attached to the computer 112. The acquired image, the barcode ID and well No. of the sample plate 1, and data about the image acquisition time and the exposure time are also simultaneously displayed on the monitor 114. The examiner removes the sample plate 1 from the stage 101 after the present image acquisition is completed, sets another sample plate 1 on the stage 101, and reads the barcode of the sample plate 1. Thus, fluorescence images of the required wells are acquired.

After a fixed period of time has elapsed, the sample plate 1 (ID:00001) whose fluorescence image was acquired earlier is set again on the stage 101, and image acquisition of all wells is performed. Fig. 13 is a fluorescence image of the rightmost well in the first row of barcode (ID:00001) after a two-hour period has elapsed. It is evident that the number of cells (cell nuclei) in the four cell groups increases. This is performed for the required number of sample plates 1. After image acquisition is completed, the examiner proceeds to verification of the acquired cell images.

The examiner starts up the cell-image analysis program on the computer 112. Then, the examiner inputs the barcode ID of the sample plate 1 to the cell-image analysis program and inputs the well number to be analyzed. It is assumed that all wells are specified at this stage.

Then, once commencement of analysis is instructed, the image analysis program loads two sets of fluorescence image data at different image acquisition times, which are already stored in the hard disk of the computer 112, into a memory, which is not shown in the drawings, from the barcode ID and well number. Then, the image analysis program extracts nucleus regions from the images.

As shown in Fig. 14, while scanning the entire image in three concentric circles with different radii (third circle 8a, second circle 8b, and first circle 8c), the image analysis program classifies them into nuclei and circular groups of pixels with a certain luminance value or greater, between the second circle 8b and the first circle 8c and not in the region of the third circle 8a. The reference numeral 8d is a cell nucleus. With this operation, the number of nuclei in the fluorescence image is counted. As a result, the number of nuclei in the fluorescence image in Fig. 12 is counted as 19, and the number of nuclei in Fig. 13 is counted as 74, and the image analysis program compares them to give (74/19) = 3.89 and displays it.

The examiner checks, for example, the degree of proliferation of the cells in the first, second, and third rows of the rightmost wells on the sample plate 1. For example, as shown in the graph in Fig. 15, if the first row indicates 3.89, the second row indicates 1.85, and the third row indicates 1.12, it is found that the effect of the compound B, serving as the drug candidate, in suppressing cell proliferation is the maximum. By performing this operation for all wells, finally averaging or the like the data in each row, and comparing the degrees of proliferation by row, it is possible to evaluate the efficacy of the compounds serving as the drug candidates. By examining the degree of proliferation of cancer cells (for example, breast cancer) using this principle, it is possible to also perform cancer pathology.

Fourth Embodiment

A fourth embodiment forming part of the present invention will be described below with reference to the drawings.
Before describing a screening method and apparatus according to this embodiment, a cell-image analysis method will be described.

Overview of Cell Analysis

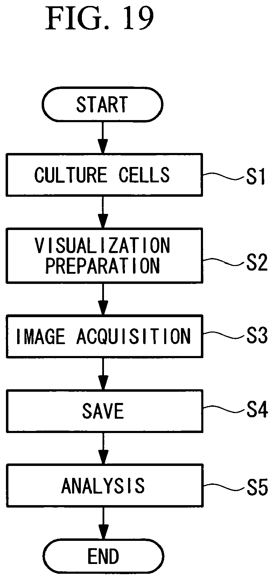

A method of automating cell-image analysis with an experimental system for observing cells, such as in drug screening, is considered.
The object of this image analysis is to provide means for comparing a plurality of cell images by specifying individual cell positions on the image and extracting a feature for each cell, and then by applying suitable statistical processing to the features.
The flow of the analysis process is described with reference to Fig. 19. First, target cells are cultured (S1). Next, suitable pretreatment such as staining is performed to prepare for visualization (S2). Cell fixation is carried out as required. These cell samples are set in an optical system such as a microscope and image acquisition is performed (S3). The acquired cell images are digitized and stored in a computer or the like (S4). These images are then processed and analyzed on the computer (S5).
Each processing step will be described next.

Pretreatment

The cells cultured by a suitable method are subjected to treatment for image acquisition.
The treatment for image acquisition includes administering a fluorescent substance. Visualization of the cells is achieved by this treatment.

Optical System, Image-acquisition System, and Data Storage

In the optical system, observation is performed by shining light of a suitable wavelength on the cells. At this time, a suitable camera is installed in the optical system and image acquisition is performed. The image-acquisition system normally digitizes the images using a CCD or the like, which are then stored in a computer or the like as data.
The cell images captured by the method described above are color or monochrome images. The images are stored as data having a width and height of, for example, $1000 \times 1000$. At each point on the image, coordinates representing the position are assigned, and each point has a luminance for each color.
The luminance at coordinates (x,y) is represented in a form such as "230", for example, and is stored as digital data.

Overview of Image Analysis

The image analysis is a method of analyzing this digital data by performing suitable processing with a software program etc. on a computer.
In particular, in image analysis using cells, cell recognition is a first objective, but normally, cell recognition is performed directly by the experimenter.
It differs according to the type of experiment, but generally, a microscope image (or the field of view of the microscope) is observed to identify, in that image, "whether or not cells exist", "if cells exist, what are the positions of the cells", "how many cells are included in the image", "how many types of cells are in the image", "how many of a specific type of cell are there", "what is the size of each cell", and so forth. The results are then recorded to provide data for experiments.
This data is subjected to statistical processing and the image acquisition conditions of each cell image and the culturing conditions of the cells are associated with each other to enable various interpretations to be conducted. In particular, by detecting differences in shape due to drugs, it is possible to perform screening of drugs and so forth, including compounds.

Compound Screening

Experiments using cells are conducted for various purposes. Here, in particular, compound screening based on cells is taken as an example, and the object and flow of the experiment is described briefly.

Cell-based compound screening is aimed at finding drugs having an effect. Therefore, this is achieved by administering suitable compounds etc. to target cells and quantifying the effects that the compounds have on the cells.

This is carried out with the aim of comparing the quantified data with cases of multiple compounds and, from a number of these compounds, finding those exerting a large influence on the cells or those with an effect.

Screening Procedure

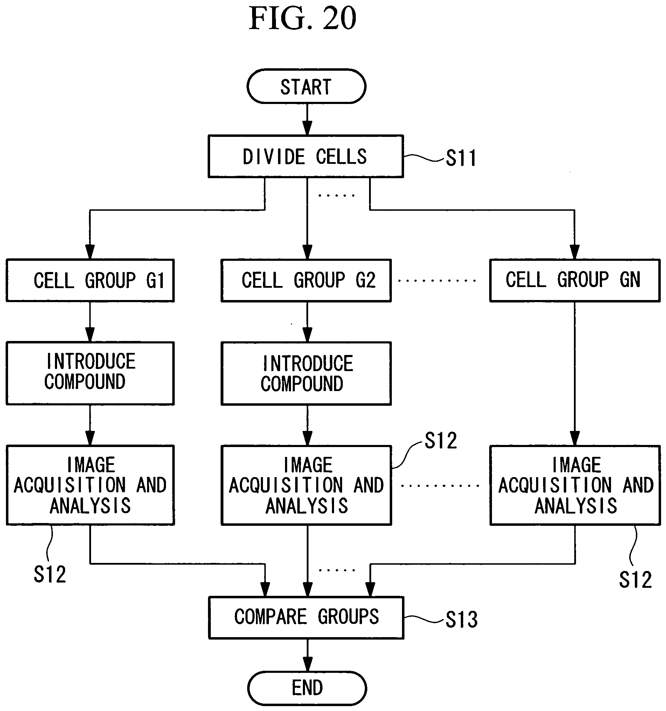

First, as shown in Fig. 20, the sample cells are normally classified into a number of groups (cell groups G1 to GN) (S11).

For example, in an experimental system to measure the effect that the compound exerts on the cells, they are divided into cell group G1 etc. to which the compound is added, and cell group GN to which it is not.

Using a suitable optical system such as a microscope, image acquisition is performed in each of these classified groups (S12).

Normally, image acquisition is performed multiple times in each group and an appropriate number of cells (around several tens) are included in each image.

Next, the cell images acquired with the above method and saved are analyzed.

Features such as the positions of cells in the image, the light-emission level and size of each cell, whether or not there is a protrusion, and so on, are extracted by analyzing the cell images.

The cell features are averaged, or their distribution is analyzed, in each group or each image, the differences between groups are detected, and they are investigated by comparing with conditions for the groups, such as whether or not the compound was introduced.

Details of Problems

Unfortunately, in actual drug screening, there is a problem in that, in many cases, the precision of evaluating the effect of the drug is not high.

Example: Definition/Selection Error in Feature Extraction

One possible reason for this is that feature extraction processing of minute shapes is itself a low-precision analysis method.

First, one reason that the precision of shape features is low may be because the definition and selection of features set as quantities representing the characteristics of the cell morphology are actually not appropriate.

An example is given in which, for instance, the cell size is extracted as a cell morphology quantity, and the feature is not compatible with the appropriate cell morphology.

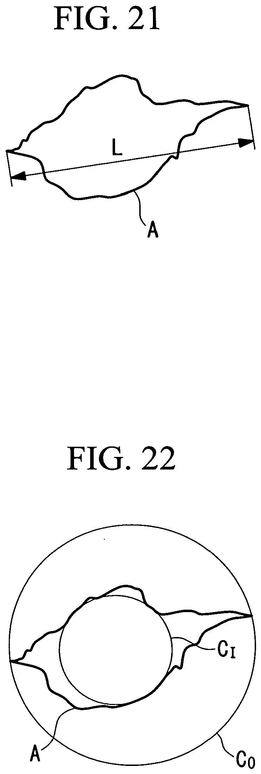

For "cell size", there is a method in which the distance L between two points separated by a maximum distance on the boundary of a cell A is normally defined as the size. According to this method, when the cell A extends in a specific direction, as shown in Fig. 21 for example, this does not mean that it suitably represents the size of the cell A.

In particular, in many cases where the shape of the cell A is distorted so as not to be close to a circle, it is difficult to accurately estimate the size of the cell A. Although the example above can be considered as corresponding to such a case, in this experimental system, the definition of the size of the cell A does not match the actual shape of the cell and is thus not suitable.

Precision Problem Occurring in Processing Methods such as Noise Removal

Image noise contained in the features due to substances other than living cells included in the cell image is a second reason for the reduced measurement accuracy of the cell features. This will be explained by taking as an example screening for finding drugs having, in particular, a protective effect on cells.

A system for applying a stimulus to culture cells such that about half of the cells die is considered as an example of such screening. A method of evaluating the protective effect on the cells based on how much the survival rate of the cells increases by the administration of a drug to the culture cell system is considered. Here, the survival rate of the cells is obtained by directly comparing the number of living cells.

The difficulty with this method is that sometimes the dead portion of the cells due the stimulus remains on the culture plate or in the culture solution.

To remove these dead cells, it is necessary to perform an operation such as washing the culture cells before microscope observation.

The time and cost are increased by adding a single treatment step like this in the actual experimental procedure. This is undesirable in drug screening where a large quantity of drug samples are to be analyzed.

In view of these circumstances, in a screening system like that described above, a high-precision analysis and processing method must be considered, assuming that living cells and dead cells coexist in the cell image.

As a method for solving the above problem, a screening method according to this embodiment will be described below.

In the screening method according to this embodiment, first, the amount of deviation of a cell A from a circular or spherical outline is used as a morphological feature representing the morphology of the cell A.

The cell A dies upon receiving the stimulus, but this process causes a continuous morphological changes in the cell A. In general, the cell A that is close to death as a result of receiving the stimulus becomes more circular due to loss of activity.

Conversely, the shape of the cell A that exhibits strong activity normally undergoes substantial deformation from a standard circular or spherical shape.

Therefore, by using the amount of deviation from a circle or sphere as the morphological feature of the cell A, it is possible to determine its viability and to estimate its degree of activity.

More specifically, extraction of morphological features is performed by the following method.

First, the boundary of the cell A is extracted by image processing, and, as shown in Fig. 22, a maximum-radius inscribed circle $C_I$ and a minimum-radius circumscribed circle $C_O$ of the boundary of the cell A are captured. Here, the radii of the circles, the area ratio of the cell region with respect to the regions included in the circles, or the like is defined as the morphological feature of the cell A.

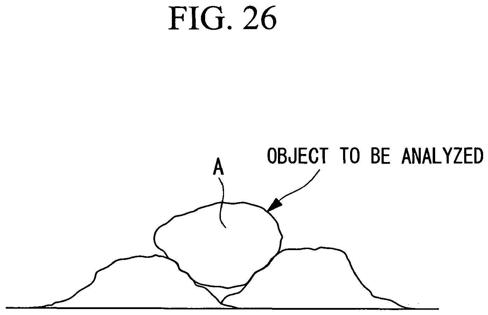

In particular, a case where cells overlap in three dimensions will now be considered (see Fig. 26). To extract and analyze the cell indicated by the arrow in this figure, it is necessary to analyze the cells in three dimensions.

When analyzing the cells A in three dimensions, an object such as the plate itself is moved slightly up and down, two-dimensional images are captured substantially continuously with the image-acquisition system, and a three-dimensional image of the cells A is constructed on the basis of this data.

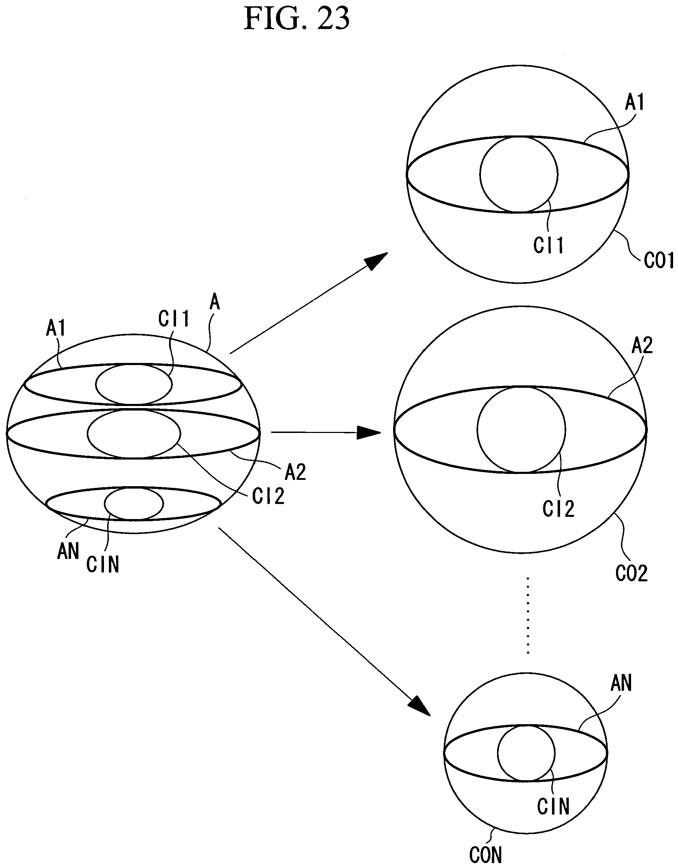

In such a case, as shown in Fig. 23, from among individual transverse sections $A_1$ to $A_N$ of the cells A, by analyzing sections passing through the center of the cells A using the method described above, it is possible to quantify the parameters described above, such as damage. In Fig. 23, reference symbols $C_{I1}$ to $C_{IN}$ indicate inscribed circles of the transverse sections $A_1$ to $A_N$ of the cells A, and reference symbols $C_{O1}$ to $C_{ON}$ indicate circumscribed circles of the transverse sections $A_1$ to $A_N$ of the cells A.

Here, the sections passing through the centers of the cells A are defined as horizontal sections where the inscribed circles $C_{I1}$ to $C_{IN}$ pass through the maximum positions in the vertical direction, for each captured two-dimensional image regarding the sections at different positions in the vertical direction of each cell A.

Features of Circles and Cells

From these morphological features, it is possible to systematically determine the sizes of the central portions of the cells A, that is, the substantial sizes of the cells A, and the deformation of the overall shape from the ratio of areas of the regions described above, and in addition, the spatial extent and so forth of the cells A from the size of the radius of the circumscribed circle. It is thus possible to efficiently and precisely represent morphological changes of the cells A.

With this method, the deformed cell A as shown in Fig. 22 for example, can be identified as the central portion contained inside the inscribed circle $C_I$, which is considered to be the substantial portion thereof. Compared with simply taking the length L, it is possible to evaluate the activity and so forth of the cell A with a value that is closer to the actual conditions.

With the screening method according to this embodiment, it is possible to determine the viability of each cell A.

By using this method, it is possible to detect a specific cellular organelle such as a protrusion, and to perform various types of classification of objects causing noise, for example, dead cells and dead fragments, or air bubbles and the like.

A specific method for dividing the image into a plurality of regions based on the method described above and classifying them into regions corresponding to the cell body, regions corresponding to cell protrusions, and regions that are not included in living cells, such as dead cells, is described below.

For example, there are many cell bodies having a morphology that is almost elliptical. In this case, the radii of the inscribed circle $C_I$ and the circumscribed circle $C_O$ can be determined since one is a few times larger than the other.

**EP 1 865 315 B1**

There are many cell components having a generally tubular structure, such as cell protrusions. In this case, however, because the inscribed circle $C_I$ is small and the circumscribed circle $C_O$ is large, the ratio of their radii is large.

In the process of cell death, the protrusions gradually contract and the cells approach a round shape overall. Therefore, for dead cells or cells that are close to death, the ratio of radii of the inscribed circle $C_I$ and the circumscribed circle $C_O$ takes a value close to 1, and in addition, the radius of the inscribed circle $C_I$ also becomes small.

With the method described above, it is possible to classify the cells based on features defined using the inscribed circle $C_I$ and the circumscribed circle $C_O$.

By interpreting these features in the following way, it is possible to directly use them as indicators of the effect of the drug.

When the cells are damaged by the stimulus they exhibit contraction. Therefore, if the deformation of the cell morphology from a circle is interpreted as damage to the cell, it is possible to use it as indicator of the protective effect of the drug.

The degree of change of the shape of the cell boundary is considered to be large when the motile activity of the cell is high. Therefore, if the deformation of the cell morphology from a circle is interpreted as originating from the cell motion, it can be used as an indicator for measuring the drug's activation on the cell.

Various categories of drug screening are possible. For example, there are drugs which protect cells, drugs which activate cells to accelerate their growth and accelerate cell division, and so forth.

Although the screening methods and cell-analysis methods for these categories of drugs differ, it is possible to apply the method of the present invention to any case.

In the case of a drug which protects the cell, the ratio of the number of living cells and the number of dead cells is a first indicator representing the efficacy. It can be measured by determining and classifying whether cells are dead or alive in the image using this method, counting the number of each type of cell, and taking their ratio.

On the other hand, for a drug having an effect of activating the cells or suppressing the activation of the cells, if there are many dead cells, it is actually not suitable as a drug and is thus ruled out as being a candidate. Here, for example, the indicator representing the activity of the cells is something that indicates how close the cell body or the cell nucleus is to elliptical. More specifically, the process described below is considered.

In this process, first, regions in the image are classified into regions that are estimated to be cells and regions that are estimated to be objects other than cells, dead cell fragments, and so forth; if the dead regions are large in comparison with the cell regions, the drug is ruled out as a candidate; the cell regions are classified into cell bodies existing at the center of the cells and constituting the majority thereof and other external regions such as cell protrusions; the degree of ellipticity of, in particular, the cell body regions is determined; and quantification is carried out for each image and each experimental condition by an averaging operation or the like to measure the respective efficacies.

Therefore, the screening method described above can be applied to various types of drug screening, such as determining the efficacy of drugs having a cell protective effect, measuring the efficacy of drugs having a cell activating effect, and so on.

In this embodiment, feature correction is carried out using image information about non-cell regions, which is not considered in normal cell image processing.

Processing Method for Images Containing Noise

In the system where the cell is subjected to damage described above, some of the cells die. In many cases, these dead cells remain in the image and are an inherent source of noise in the system, thus causing a decline in the precision of the cell feature analysis.

In normal analysis, image information about non-cell regions such as dead cells is normally removed by suitable function processing and so forth.

In the screening method according to this embodiment on the other hand, feature correction is performed using this image information which is normally removed.

For example, in the system described above where the number of cells is counted to define an indicator of the protective effect on the cells, the number of cells is not always strictly controlled in advance. Therefore, a method in which the overall number of cells is averaged by observing many cell images in the same category, that is, under similar conditions, to suppress variations in the number of cells in the image can be used.

However, averaging and correcting using such a method in cell screening increases the analysis time for the experiment, and therefore, it is not a desirable method of correction.

In this embodiment, by dividing the total number of cells by the number of dead cells, it is possible to accurately estimate the ratio of dead cells or living cells with respect to the number of cells before applying the stimulus.

In addition, when evaluating the damage to cells using, for example, deformation of the morphology, not just correction of the number in this way, the total number of dead cells and the total area of the dead cell region are both features corresponding to the cell damage.

Thus, by comparing or averaging them, the amount of damage to the cells can be corrected, and it is possible to obtain a value that is closer to the actual cell damage.

As described above, by quantifying image information about non-cell regions and performing screening using it as additional information for the results of image analysis on only the cells, the precision of the cell analysis can be expected to increase.

In particular, when performing screening using nerve cells, screening is sometimes difficult because of their more peculiar morphology compared to normal cells.

For example, in the case of a system in which the total length of nerve cell protrusions having a tubular structure is defined as a feature representing the morphology, the total length of the protrusions exhibits a large variation from cell to cell, is easily affected by drugs or the like, or is difficult to detect in image analysis compared to the cell body. Therefore, in many cases, the results of the analysis processing contain large errors.

In contrast to this case, by using information about dead cells and so forth, two indicators of cell damage are obtained. It is thus possible to derive more suitable values, and the overall screening precision can thus be expected to increase.

In an experimental system like this for observing cells that are subjected to suitable treatment, such as the administration of a drug, in many cases, not only the type and amount of the drug but also the culture conditions are important factors governing the results.

Cells are generally considered to exhibit interactions, and these interactions occur not just between cells of the same type, but also between cells of multiple different types.

When observing a sample in which multiple types of cells are cultured together, first, it is necessary to perform processing for distinguishing between the multiple types of cells.

Possible methods for classifying multiple types of cells include methods of assessing the type from something qualitative, such as differences in the morphology of each cell or differences in staining, or from quantitative differences, such as fluorescence intensity.

After performing these processes, feature extraction and so forth is performed with the same procedure as analysis conducted on a single type of cell, described above, and changes etc. due to drugs are quantified.

By classifying the cells on the sample into categories, such as cell type, and recording changes etc. in the features for each category, it is possible to perform more detailed data interpretation, and the discovery of effects can be expected by, for example, searching for effective drugs.

When culturing multiple types of cells, it is expected that the results will differ depending on the mixing ratios of the cells; therefore, this information, mixing ratios, and so forth is also used in combination.

By comparing not only the ratios of the cells but also differences in the responses of each type of cell to drugs, it should be possible, for example, to search for drugs more accurately.

The method of using this information is the same as, for example, the method of correcting using dead cells.

For example, in a system including two types of cells, cell group G1 and cell group G2, it is assumed that results are obtained indicating that 60% of the cell group G1 dies and 10% of the cell group G2 dies by applying a fixed stimulus after administering a drug. This case can be interpreted as the drug having a role of selectively protecting the cell group G2.

In general, by comparing the results for the different cell types in this way, it is expected that more detailed analysis will be possible.

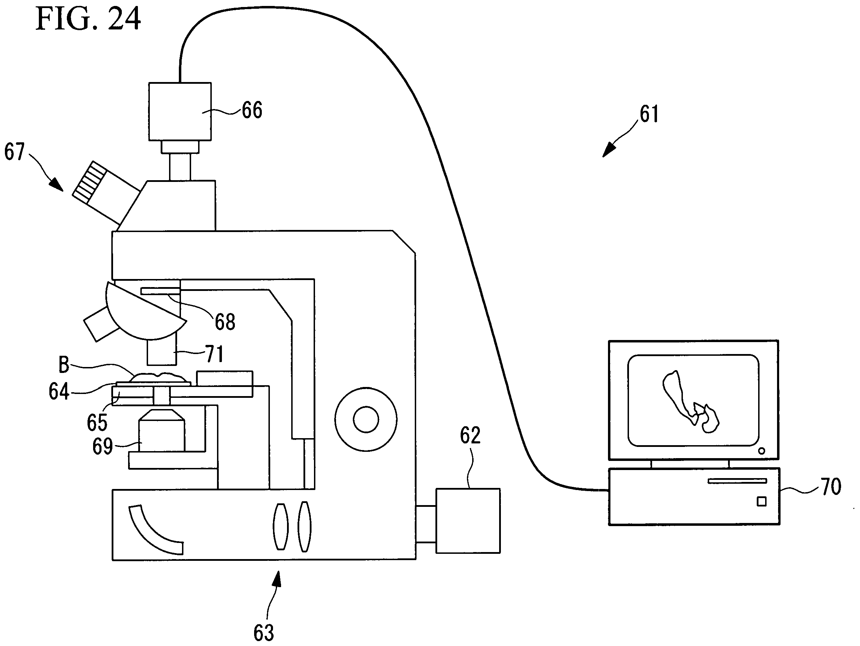

Next, a screening apparatus according to a fourth embodiment not forming part of the present invention will be described with reference to Fig. 24.

Fig. 24 shows the overall configuration of a screening apparatus 61 according to this embodiment. The screening apparatus 61 according to this embodiment includes an illumination optical system 67 (illumination unit) 63 including a light source 62 for generating excitation light; a positioning stage 65 for mounting a plate 64 on which a sample B, including plant or animal cells to be observed, is disposed; a light-receiving optical system including a CCD camera (image-information inputting unit) 66 for acquiring fluorescence emitted from the sample B; an image processing unit for processing the images captured by the CCD camera 66 to extract morphological features; and a screening unit for performing drug screening using the extracted morphological features.

A condenser lens 69 for radiating the excitation light from the light source 62 onto the sample B is provided in the illumination optical system 63.

The positioning stage 65 can move the plate 64 so as to focus at the sample B.

The light-receiving optical system 67 is provided with a filter 68 having an optical characteristic allowing transmission of fluorescence of a specific wavelength band. The filter 68 is replaceable so that it can be replaced according to the wavelength band to be transmitted.

A general-purpose processing device which operates according to a computer program, such as a computer (screening unit, evaluation unit, image processing unit, image-information processing unit) 70, is used as the image processing unit and the screening unit. A computer program for making the computer 70 function as the image processing unit, for example, a computer program for realizing functions such as cell-center extraction processing, center-point examination processing, cell-boundary extraction processing, feature extraction processing for individual cells, and total-cell-number extraction processing, is installed in the computer 70. In addition, in order to make it function as the screening unit, a computer program for realizing the screening method according to this embodiment is also installed in the computer 70.

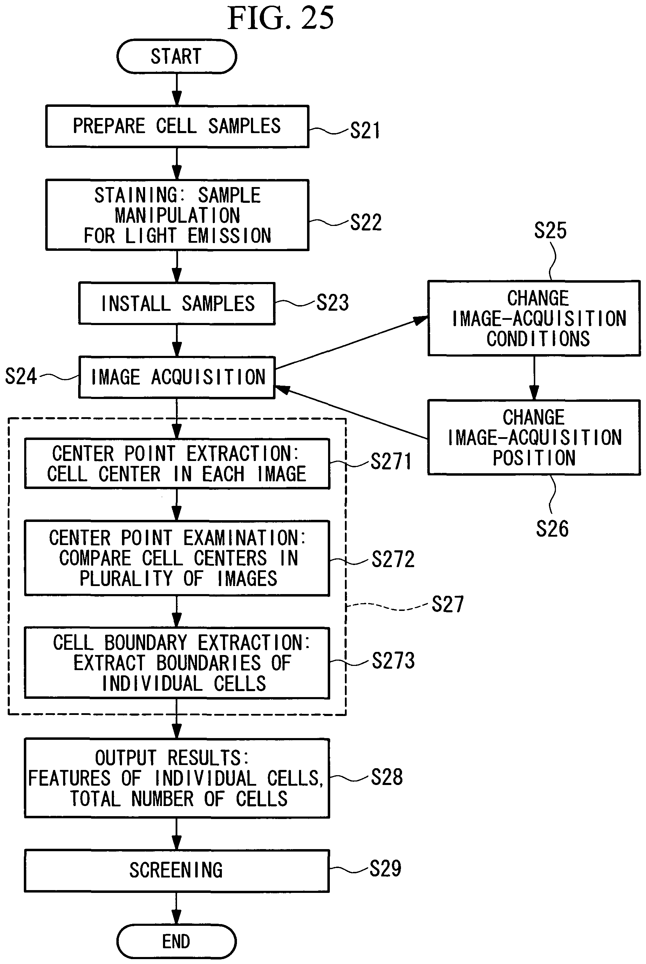

To perform drug screening using the screening apparatus 61 according to this embodiment, as shown in Fig. 25, first, the sample B, such as cell samples, is placed on the plate 64. In a cell-sample preparation step S21, the sample B to be observed under various conditions is prepared. Then, in a sample manipulation step S22 for staining and fluorescence, a suitable staining method and manipulation for causing fluorescence are performed on the sample B. Then, in a sample installation step S23, the plate 64 prepared in this manner is fixed to the positioning stage 65, observation-position alignment and focus-position alignment are performed by operating the positioning stage 65, and the excitation light from the light source 62 is radiated toward the cell sample B.

Because fluorescence is emitted from the sample B irradiated with the excitation light, the emitted fluorescence is collected by an objective lens 71, and only fluorescence of a predetermined wavelength band is selected by the filter 68 and is acquired by the CCD camera 66 in the image-acquisition step S24. Image acquisition is performed by changing various image-acquisition conditions (S25) as required, such as replacing the filter 68 while keeping the sample B fixed. Alternatively, image acquisition is performed by changing the image-acquisition position (S26) as required.

The fluorescence image information captured by the CCD camera is sent to the image processing unit, where an image processing step S27 is performed.

In the image processing step S27, first, the points at the centers of individual cells in each image are extracted (S271). The extracted center points are examined, and suitable cell center points from which unsuitable points have been eliminated are extracted as the center points of the cells (S272). Then, the most suitable cell boundaries surrounding these cell center points are extracted (S273). Then, by using the cell boundaries obtained, the screening method described above, that is to say, extraction of the morphological features of individual cells and calculation of the total number of cells, is performed (S28), and drug screening is realized on the basis thereof (S29).

Thus, the screening method and apparatus according to this embodiment afford an advantage in that it is possible to realize high-precision drug screening using cells.

**Claims**

1. A cell-image analysis method comprising:

   an image-acquisition step of capturing a plurality of cell images under different image-acquisition conditions;
   a cell position extracting step of extracting cell positions on the basis of the plurality of cell images;
   a feature extracting step of extracting features on the cells extracted in the cell position extracting step; and
   **characterized by**
   a statistics processing step of performing statistical analysis with the features extracted in the feature extracting step serving as parameters and verifying correlations of the features to select and/or design parameters appropriate for cell evaluation.

2. A cell-image analysis method according to Claim 1, further comprising a step of evaluating effectiveness of a compound administered to the cells, using the parameters selected and/or designed in the statistics processing step.

3. A cell-image analysis method according to Claim 1, wherein in the statistics processing step, weightings of the parameters are adjusted on the basis of the correlations.

4. A cell-image analysis method according to Claim 2 or Claim 3, wherein in the statistics processing step, measurement accuracy or measurement reliability of the parameters is evaluated on the basis of the correlation and measurement results.

5. A cell-image analysis method according to Claim 4, wherein in the statistics processing step, the parameters that most effectively represent a change due to conditions of a measurement system are selected on the basis of the evaluation of the measurement accuracy or the measurement reliability of the parameters.

6. A cell-image analysis method according to Claim 5, wherein in the statistics processing step, the parameter that is the most effective indicator in cell evaluation is selected on the basis of the selected parameters.

7. A cell-image analysis method according to Claim 1, wherein in the statistics processing step, the features of the cells are analyzed using position information of each cell.

8. A cell-image analysis method according to Claim 7, wherein the position information includes local cell density, distance to nearest cell, and information about whether or not cells are in contact.

9. A cell-image analysis apparatus comprising:

an image-acquisition unit for capturing a plurality of cell images under different image-acquisition conditions;
a cell position extracting unit for extracting cell positions on the basis of the plurality of cell images;
a feature extracting unit for extracting features on the cells extracted by the cell position extracting unit; and **characterized by**
a statistics processing unit for performing statistical analysis with the features extracted by the feature extracting unit serving as parameters,
wherein the statistics processing unit verifies correlations of the features to select and/or design parameters appropriate for cell evaluation.

10. A cell-image analysis apparatus according to Claim 9, wherein the statistics processing unit evaluates the effectiveness of a compound administered to the cells using the selected and/or designed parameters.

11. A cell-image analysis apparatus according to Claim 9, wherein the statistics processing unit changes weightings of the parameters on the basis of the correlation.

12. A cell-image analysis apparatus according to any one of Claims 9 to 11, wherein the statistics processing unit evaluates measurement accuracy or measurement reliability of the parameters on the basis of the correlations and measurement results.

13. A cell-image analysis apparatus according to Claim 12, wherein the statistics processing unit selects parameter that most effectively represents a change due to conditions of a measurement system on the basis of the evaluation of the measurement accuracy or the measurement reliability of the parameters.

14. A cell-image analysis apparatus according to Claim 13, wherein the statistics processing unit selects the parameter that is the most effective indicator in cell evaluation on the basis of the selected parameters.

15. A cell-image analysis apparatus according to Claim 9, wherein the statistics processing unit analyzes the features of cells using position information of each cell.

16. A cell-image analysis apparatus according to Claim 15, wherein the position information includes local cell density, distance to nearest cell, and information about whether or not cells are in contact.

**Patentansprüche**

1. Zellbildanalyseverfahren, umfassend:

einen Bilderfassungsschritt eines Aufnehmens einer Vielzahl von Zellbildern unter verschiedenen Bilderfassungsbedingungen;
einen Zellpositionsextraktionsschritt eines Extrahierens von Zellpositionen auf der Basis der Vielzahl von Zellbildern;
einen Merkmalsextraktionsschritt eines Extrahierens von Merkmalen auf den Zellen, die in dem Zellpositionsextraktionsschritt extrahiert wurden; **gekennzeichnet durch**
einen Statistikverarbeitungsschritt eines Ausführens einer statistischen Analyse mit den in dem Merkmalsextraktionsschritt extrahierten Merkmalen, die als Parameter dienen, und eines Verifizierens von Korrelationen der Merkmale zum Auswählen und/oder Konstruieren von Parametern, die angemessen zur Zellevaluation sind.

2. Zellbildanalyseverfahren nach Anspruch 1, weiter umfassend einen Schritt eines Evaluierens einer Effektivität einer Verbindung, die den Zellen verabreicht wurde, durch Verwenden der Parameter, die ausgewählt wurden und/oder in dem Statistikverarbeitungsschritt konstruiert wurden.

**3.** Zellbildanalyseverfahren nach Anspruch 1, wobei in dem Statistikverarbeitungsschritt Gewichtungen der Parameter auf der Basis der Korrelationen angepasst werden.

**4.** Zellbildanalyseverfahren nach Anspruch 2 oder Anspruch 3, wobei in dem Statistikverarbeitungsschritt eine Messgenauigkeit oder Messverlässlichkeit der Parameter auf der Basis der Korrelation und von Messergebnissen evaluiert wird.

**5.** Zellbildanalyseverfahren nach Anspruch 4, wobei in dem Statistikverarbeitungsschritt die Parameter, die am meisten effektiv eine Änderung aufgrund Bedingungen eines Messsystems darstellen, auf der Basis der Evaluierung der Messgenauigkeit oder der Messverlässlichkeit der Parameter ausgewählt werden.

**6.** Zellbildanalyseverfahren nach Anspruch 5, wobei in dem Statistikverarbeitungsschritt der Parameter, der der am meisten effektive Indikator in einer Zellevaluation ist, auf der Basis der ausgewählten Parameter ausgewählt wird.

**7.** Zellbildanalyseverfahren nach Anspruch 1, wobei in dem Statistikverarbeitungsschritt die Merkmale der Zelle durch Verwenden von Positionsinformationen jeder Zelle analysiert werden.

**8.** Zellbildanalyseverfahren nach Anspruch 7, wobei die Positionsinformationen eine lokale Zelldichte, eine Distanz zur nächsten Zelle und Informationen darüber beinhalten, ob Zellen in Kontakt sind oder nicht.

**9.** Zellbildanalysevorrichtung, umfassend:

eine Bilderfassungseinheit zum Aufnehmen einer Vielzahl von Bildern unter verschiedenen Bilderfassungsbedingungen;
eine Zellpositionsextraktionseinheit zum Extrahieren von Zellpositionen auf der Basis der Vielzahl von Zellbildern;
eine Merkmalsextraktionseinheit zum Extrahieren von Merkmalen auf den Zellen, welche durch die Zellextraktionseinheit extrahiert wurden; und **gekennzeichnet durch**
eine Statistikverarbeitungseinheit zum Ausführen einer statistischen Analyse mit den als Parameter dienenden Merkmalen, die durch die Merkmalsextraktionseinheit extrahiert wurden,
wobei die Statistikverarbeitungseinheit Korrelationen der Merkmale zum Auswählen und/oder Konstruieren von Parametern verifiziert, die zur Zellevaluation angemessen sind.

**10.** Zellbildanalysevorrichtung nach Anspruch 9, wobei die Statistikverarbeitungseinheit die Effektivität einer Verbindung evaluiert, die den Zellen verabreicht wurde, durch Verwenden des ausgewählten und/oder konstruierten Parameters.

**11.** Zellbildanalysevorrichtung nach Anspruch 9, wobei die Statistikverarbeitungseinheit Gewichtungen der Parameter auf der Basis der Korrelation ändert.

**12.** Zellbildanalysevorrichtung nach einem der Ansprüche 9 bis 11, wobei die Statistikverarbeitungseinheit eine Messgenauigkeit oder Messverlässlichkeit der Parameter auf der Basis der Korrelationen und Messergebnisse evaluiert.

**13.** Zellbildanalysevorrichtung nach Anspruch 12, wobei die Statistikverarbeitungseinheit Parameter, die am meisten effektiv eine Änderung aufgrund Bedingungen eines Messsystems darstellen, auf der Basis der Evaluierung der Messgenauigkeit oder der Messverlässlichkeit der Parameter auswählt.

**14.** Zellbildanalysevorrichtung nach Anspruch 13, wobei die Statistikverarbeitungseinheit den Parameter, der der am meisten effektive Indikator in einer Zellevaluation ist, auf der Basis der ausgewählten Parameter auswählt.

**15.** Zellbildanalysevorrichtung nach Anspruch 9, wobei die Statistikverarbeitungsvorrichtung die Merkmale der Zelle durch Verwenden von Positionsinformationen jeder Zelle analysiert.

**16.** Zellbildanalysevorrichtung nach Anspruch 15, wobei die Positionsinformationen eine lokale Zelldichte, eine Distanz zur nächsten Zelle und Informationen darüber beinhalten, ob Zellen in Kontakt sind oder nicht.

**Revendications**

1. Procédé d'analyse d'images de cellules comprenant :

   une étape d'acquisition d'images consistant à capturer une pluralité d'images de cellules dans différentes conditions d'acquisition d'images ;
   une étape d'extraction de positions de cellules consistant à extraire des positions de cellules sur la base de la pluralité d'images de cellules ;
   une étape d'extraction de caractéristique consistant à extraire des caractéristiques sur les cellules extraites dans l'étape d'extraction de positions de cellules ; et **caractérisé par**
   une étape de traitement de statistiques consistant à réaliser une analyse statistique avec les caractéristiques extraites dans l'étape d'extraction de caractéristique servant en tant que paramètres et à vérifier des corrélations des caractéristiques pour sélectionner et/ou concevoir des paramètres appropriés pour une évaluation de cellules.

2. Procédé d'analyse d'images de cellules selon la revendication 1, comprenant en outre une étape consistant à évaluer l'efficacité d'un composé administré aux cellules, à l'aide des paramètres sélectionnés et/ou conçus dans l'étape de traitement de statistiques.

3. Procédé d'analyse d'images de cellules selon la revendication 1, dans lequel, dans l'étape de traitement de statistiques, des pondérations des paramètres sont ajustées sur la base des corrélations.

4. Procédé d'analyse d'images de cellules selon la revendication 2 ou la revendication 3, dans lequel, dans l'étape de traitement de statistiques, une précision de mesure ou une fiabilité de mesure des paramètres est évaluée sur la base de la corrélation et des résultats de mesure.

5. Procédé d'analyse d'images de cellules selon la revendication 4, dans lequel, dans l'étape de traitement de statistiques, les paramètres qui représentent le plus efficacement un changement dû à des conditions d'un système de mesure sont sélectionnés sur la base de l'évaluation de la précision de mesure ou de la fiabilité de mesure des paramètres.

6. Procédé d'analyse d'images de cellules selon la revendication 5, dans lequel, dans l'étape de traitement de statistiques, le paramètre qui est l'indicateur le plus efficace lors d'une évaluation de cellules est sélectionné sur la base des paramètres sélectionnés.

7. Procédé d'analyse d'images de cellules selon la revendication 1, dans lequel, dans l'étape de traitement de statistiques, les caractéristiques des cellules sont analysées à l'aide d'informations de position de chaque cellule.

8. Procédé d'analyse d'images de cellules selon la revendication 7, dans lequel les informations de position comprennent une densité cellulaire locale, une distance par rapport à la cellule la plus proche et des informations concernant le point de savoir si des cellules sont ou non en contact.

9. Appareil d'analyse d'images de cellules comprenant :

   une unité d'acquisition d'images pour capturer une pluralité d'images de cellules dans différentes conditions d'acquisition d'images ;
   une unité d'extraction de positions de cellules pour extraire des positions de cellules sur la base de la pluralité d'images de cellules ;
   une unité d'extraction de caractéristique pour extraire des caractéristiques sur les cellules extraites par l'unité d'extraction de positions de cellules ; et **caractérisé par**
   une unité de traitement de statistiques pour réaliser une analyse statistique avec les caractéristiques extraites par l'unité d'extraction de caractéristique servant en tant que paramètres,
   l'unité de traitement de statistiques vérifiant des corrélations des caractéristiques pour sélectionner et/ou concevoir des paramètres appropriés pour une évaluation de cellules.

10. Appareil d'analyse d'images de cellules selon la revendication 9, dans lequel l'unité de traitement de statistiques évalue l'efficacité d'un composé administré aux cellules, à l'aide des paramètres sélectionnés et/ou conçus.

**11.** Appareil d'analyse d'images de cellules selon la revendication 9, dans lequel l'unité de traitement de statistiques change des pondérations des paramètres sur la base de la corrélation.

**12.** Appareil d'analyse d'images de cellules selon l'une quelconque des revendications 9 à 11, dans lequel l'unité de traitement de statistiques évalue une précision de mesure ou une fiabilité de mesure des paramètres sur la base des corrélations et des résultats de mesure.

**13.** Appareil d'analyse d'images de cellules selon la revendication 12, dans lequel l'unité de traitement de statistiques sélectionne un paramètre qui représente le plus efficacement un changement dû à des conditions d'un système de mesure sur la base de l'évaluation de la précision de mesure ou de la fiabilité de mesure des paramètres.

**14.** Appareil d'analyse d'images de cellules selon la revendication 13, dans lequel l'unité de traitement de statistiques sélectionne le paramètre qui est l'indicateur le plus efficace lors d'une évaluation de cellules sur la base des paramètres sélectionnés.

**15.** Appareil d'analyse d'images de cellules selon la revendication 9, dans lequel l'unité de traitement de statistiques analyse les caractéristiques de cellules à l'aide d'informations de position de chaque cellule.

**16.** Appareil d'analyse d'images de cellules selon la revendication 15, dans lequel les informations de position comprennent une densité cellulaire locale, une distance par rapport à la cellule la plus proche et des informations concernant le point de savoir si des cellules sont ou non en contact.

# FIG. 1

# FIG. 2

```
┌─────────────────────────┐
│   ACQUIRE PLURALITY     │
│      OF IMAGES          │── S1
└─────────────────────────┘
            │
            ▼
┌─────────────────────────┐
│   EXTRACT CENTER OF     │
│    EACH CELL IMAGE      │── S2
└─────────────────────────┘
            │
            ▼
┌─────────────────────────┐
│  COMPARE PLURALITY OF   │
│   IMAGES  TO EXTRACT    │── S3
│     CELL CENTERS        │
└─────────────────────────┘
            │
            ▼
┌─────────────────────────┐
│  EXTRACT BOUNDARIES OF  │── S4
│    INDIVIDUAL CELLS     │
└─────────────────────────┘
            │
            ▼
┌─────────────────────────┐
│   OUTPUT FEATURES OF    │
│  INDIVIDUAL CELLS AND   │── S5
│  TOTAL NUMBER OF CELLS  │
└─────────────────────────┘
```

# FIG. 3

(a)

(b)

FIG. 4

FIG. 5

(a)

SIZE

(b)

SIZE

FIG. 6

X1

X2

# FIG. 7

EP 1 865 315 B1

# FIG. 8

# FIG. 9

# FIG. 10

# FIG. 11

5-A

5-C

5-B

5-D

## FIG. 12

## FIG. 13

# FIG. 14

# FIG. 15

# FIG. 16

```
                START
                  │
                  ▼
        ┌──────────────────────┐
        │   ADMINISTER DRUG    │
        │ CANDIDATE COMPOUNDS  │
        │  TO SAMPLE PLATE     │
        └──────────────────────┘
                  │
                  ▼
        ┌──────────────────────┐
        │  SET  SAMPLE PLATE   │◄─────┐
        │     ON STAGE         │      │
        └──────────────────────┘      │
                  │                   │
                  ▼                   │
        ┌──────────────────────┐      │
        │  READ BARCODE ID OF  │      │
        │    SAMPLE PLATE      │      │
        └──────────────────────┘      │
                  │                   │
                  ▼                   │
        ┌──────────────────────┐      │
        │  SPECIFY WELL TO BE   │      │
        │ ACQUIRED AND INSTRUCT │      │
        │    COMMENCEMENT       │      │
        │    OF OPERATION       │      │
        └──────────────────────┘      │
                  │                   │
                  ▼                   │
        ┌──────────────────────┐      │
        │    OBSERVATION,       │◄──┐  │
        │ IMAGE-ACQUISITION,    │   │  │
        │    STORAGE            │   │  │
        └──────────────────────┘   │  │
                  │                │  │
                  ▼                │  │
            HAS IMAGE              │  │
          ACQUISITION BEEN   NO ───┘  │
          COMPLETED FOR               │
            ALL WELLS?                │
                  │ YES              │
                  ▼                  │
            HAS IMAGE                │
          ACQUISITION BEEN   NO ─────┘
          COMPLETED FOR
            ALL PLATES?
                  │ YES
                  └────────────┐
```

```
        ┌──────────────────────┐
        │ SET SAMPLE PLATE ON  │
        │ STAGE A PREDETERMINED │◄────┐
        │  DURATION AFTER       │     │
        │ ADMINISTERING DRUG    │     │
        │ CANDITATE COMPOUNDS   │     │
        └──────────────────────┘     │
                  │                  │
                  ▼                  │
        ┌──────────────────────┐     │
        │  READ BARCODE ID OF  │     │
        │    SAMPLE PLATE      │     │
        └──────────────────────┘     │
                  │                  │
                  ▼                  │
        ┌──────────────────────┐     │
        │  SPECIFY WELL TO BE   │     │
        │ ACQUIRED AND INSTRUCT │     │
        │    COMMENCEMENT       │     │
        │    OF OPERATION       │     │
        └──────────────────────┘     │
                  │                  │
                  ▼                  │
        ┌──────────────────────┐     │
        │    OBSERVATION,       │◄─┐  │
        │ IMAGE-ACQUISITION,    │  │  │
        │    STORAGE            │  │  │
        └──────────────────────┘  │  │
                  │               │  │
                  ▼               │  │
            HAS                   │  │
        IMAGE-ACQUISITION   NO ───┘  │
        BEEN COMPLETED FOR           │
            ALL WELLS?               │
                  │ YES            │
                  ▼                │
            HAS                   │
        IMAGE-ACQUISITION   NO ───┘
        BEEN COMPLETED FOR
            ALL PLATES?
                  │ YES
                  ▼
                END
```

# FIG. 17

```
╭─────────────────────────────╮
│   INITIATE ANALYSIS PROGRAM  │
╰─────────────────────────────╯
              │
              ▼
┌─────────────────────────────────┐
│          INPUT BARCODE ID        │
│     INPUT WELL NO. AND INSTRUCT  │ ◄──┐
│      COMMENCEMENT OF ANALYSIS    │    │
└─────────────────────────────────┘    │
              │                         │
              ▼                         │
┌─────────────────────────────────┐    │
│   BASED ON BARCODE ID, COMPUTER  │    │
│      READS IMAGES AND            │    │
│      IMAGE-ACQUISITION DATA      │    │
│   ACQUIRED DIRECTLY AFTER        │    │
│   ADMINISTERING                  │    │
│   THE COMPOUND AND AFTER ELAPSE  │    │
│   OF TIME PERIOD FROM HARD DISK  │    │
└─────────────────────────────────┘    │
              │                         │
              ▼                         │
┌─────────────────────────────────┐    │
│   SUBJECT IMAGES ACQUIRED        │    │
│   DIRECTLY AFTER                 │    │
│   ADMINISTERING THE COMPOUND AND │    │
│   AFTER ELAPSE OF TIME PERIOD TO │    │
│   BINARIZATION PROCESSING        │    │
└─────────────────────────────────┘    │
              │                         │
              ▼                         │
┌─────────────────────────────────┐    │
│   COUNT NUMBERS OF WHITE-LEVEL   │    │
│   PIXELS                         │    │
│   IN BINARIZED IMAGES ACQUIRED   │    │
│   DIRECTLY                       │    │
│   AFTER ADMINISTERING THE        │    │
│   COMPOUND AND                   │    │
│   AFTER ELAPSE OF TIME PERIOD    │    │
└─────────────────────────────────┘    │
              │                         │
              ▼                         │
┌─────────────────────────────────┐    │
│   CALCULATE DEGREE OF            │    │
│   PROLIFERATION                  │    │
│   FROM NUMBERS OF WHITE-LEVEL    │    │
│   PIXELS                         │    │
│   IN BINARIZED IMAGES ACQUIRED   │    │
│   DIRECTLY                       │    │
│   AFTER ADMINISTERING THE        │    │
│   COMPOUND                       │    │
│   AND AFTER ELAPSE OF TIME       │    │
│   PERIOD                         │    │
└─────────────────────────────────┘    │
              │                         │
              ▼                         │
┌─────────────────────────────────┐    │
│          GRAPH RESULTS           │    │
└─────────────────────────────────┘    │
              │                         │
              ▼                         │
         ╱──────────╲                   │
        ╱ HAS ANALYSIS ╲     NO         │
       ⟨ OF THE REQUIRED ⟩─────────────┘
        ╲ PLATES BEEN   ╱
         ╲ COMPLETED?  ╱
          ╲──────────╱
              │ YES
              ▼
        ╭──────────╮
        │   END    │
        ╰──────────╯
```

# FIG. 18

( INITIATE ANALYSIS PROGRAM )

INPUT BARCODE ID
INPUT WELL NO. AND INSTRUCT
COMMENCEMENT OF ANALYSIS

BASED ON BARCODE ID, COMPUTER READS
IMAGES AND IMAGE-ACQUISITION DATA
ACQUIRED DIRECTLY AFTER ADMINISTERING
THE COMPOUND AND AFTER ELAPSE OF
TIME PERIOD FROM HARD DISK

EXTRACT NUCLEI FROM IMAGES
ACQUIRED DIRECTLY AFTER
ADMINISTERING THE COMPOUND
AND AFTER ELAPSE OF TIME PERIOD

COUNT NUMBERS OF NUCLEI IN BINARIZED
IMAGES ACQUIRED DIRECTLY AFTER
ADMINISTERING THE COMPOUND AND
AFTER ELAPSE OF TIME PERIOD

CALCULATE DEGREE OF PROLIFERATION
FROM NUMBERS OF NUCLEI IN BINARIZED
IMAGES ACQUIRED DIRECTLY AFTER
ADMINISTERING THE COMPOUND AND
AFTER ELAPSE OF TIME PERIOD

GRAPH RESULTS

HAS ANALYSIS
OF THE REQUIRED PLATES
BEEN COMPLETED?                    NO

YES

( END )

# FIG. 19

START

↓

CULTURE CELLS — S1

↓

VISUALIZATION
PREPARATION — S2

↓

IMAGE ACQUISITION — S3

↓

SAVE — S4

↓

ANALYSIS — S5

↓

END

# FIG. 20

# FIG. 21

# FIG. 22

# FIG. 23

FIG. 24

EP 1 865 315 B1

# FIG. 25

```
                    ┌──────────┐
                    │  START   │
                    └────┬─────┘
                         ▼
        ┌─────────────────────────────┐
        │    PREPARE CELL SAMPLES      │───S21
        └──────────────┬──────────────┘
                       ▼
        ┌─────────────────────────────┐
        │     STAINING: SAMPLE         │
        │      MANIPULATION            │───S22
        │   FOR LIGHT EMISSION         │
        └──────────────┬──────────────┘
                       ▼
        ┌─────────────────────────┐                      ┌──────────────────────────┐
        │    INSTALL SAMPLES      │───S23        S25──────│       CHANGE             │
        └──────────────┬──────────┘                      │ IMAGE-ACQUISITION        │
                       ▼                                  │   CONDITIONS             │
 S24──┌─────────────────────────┐                        └────────────┬─────────────┘
      │    IMAGE ACQUISITION     │◄──────────                          ▼
      └─────────────┬────────────┘                        ┌──────────────────────────┐
                    ▼                                      │       CHANGE             │
 ┌ ─ ─ ─ ─ ─ ─ ─ ─ ─ ─ ─ ─ ─ ─ ─ ─ ┐                     │ IMAGE-ACQUISITION        │
 │  ┌───────────────────────────┐   │                     │    POSITION              │
 │  │ CENTER POINT EXTRACTION:   │───S271                  └────────────┬─────────────┘
 │  │ CELL CENTER IN EACH IMAGE  │   │                                  │
 │  └─────────────┬─────────────┘   │                                 S26
 │                ▼                  │
 │  ┌───────────────────────────┐   │
 │  │ CENTER POINT EXAMINATION:  │   │
 │  │ COMPARE CELL CENTERS IN    │───S272
 │  │  PLURALITY OF IMAGES       │   │
 │  └─────────────┬─────────────┘   │────S27
 │                ▼                  │
 │  ┌───────────────────────────┐   │
 │  │ CELL BOUNDARY EXTRACTION:  │   │
 │  │   EXTRACT BOUNDARIES OF    │───S273
 │  │    INDIVIDUAL CELLS        │   │
 │  └─────────────┬─────────────┘   │
 └ ─ ─ ─ ─ ─ ─ ─ ─┼─ ─ ─ ─ ─ ─ ─ ─ ┘
                  ▼
        ┌─────────────────────────────┐
        │      OUTPUT RESULTS:         │
        │ FEATURES OF INDIVIDUAL CELLS,│───S28
        │    TOTAL NUMBER OF CELLS     │
        └──────────────┬──────────────┘
                       ▼
        ┌─────────────────────────────┐
        │         SCREENING            │───S29
        └──────────────┬──────────────┘
                       ▼
                 ┌──────────┐
                 │   END    │
                 └──────────┘
```

FIG. 26

A

OBJECT TO BE ANALYZED

**EP 1 865 315 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001269195 A

- US 6743576 B1